# EUROPEAN PATENT SPECIFICATION

(11) **EP 3 078 365 B1**
(45) Date of publication and mention of the grant of the patent: **04.03.2020**
(21) Application number: 16164501.5
(22) Date of filing: 08.04.2016
(51) Int. Cl.: A61K 8/39, A61K 8/44, A61K 8/60, A61Q 13/00, A61Q 19/00, A61Q 15/00, A61K 8/86

(54) **SOLUBILIZING AGENTS AND AQUEOUS COMPOSITIONS COMPRISING THEM**
LÖSUNGSVERMITTLER UND WÄSSRIGE ZUSAMMENSETZUNGEN DAMIT
AGENTS SOLUBILISANTS ET COMPOSITIONS AQUEUSES LES COMPRENANT

(30) Priority: 10.04.2015 EP 15305534
(43) Date of publication of application: 12.10.2016
(73) Proprietor: Daito Kasei Industries France, 95150 Taverny (FR)
(72) Inventor: SEU-SALERNO, Martine, 92200 NEUILLY SUR SEINE (FR); FILLARDET, Laurence, 95300 PONTOISE (FR); NODA, Hisatoshi, 95740 FREPILLON (FR); ISHIMURA, Ryota, 95740 FREPILLON (FR); NISHIMOTO, Kenji, 95740 FREPILLON (FR)
(74) Representative: Lavoix

(56) References cited:
- WO-A1-2005/039642
- WO-A1-2014/076136
- WO-A1-2015/054135
- US-A- 5 789 371
- US-A1- 2014 121 174
- DATABASE GNPD [Online] MINTEL; 1 November 2014 (2014-11-01), Anonymous: "GNPD - Active Surge Eye Cream", XP055213131, retrieved from Mintel accession no. 2769661 Database accession no. 2769661
- CASTRO M J L ET AL: "SYNERGISTIC EFFECTS OF ALKYL GLUCOSIDES AND NONIONIC SUGAR-BASED GEMINI SURFACTANTS//SYNERGISTISCHE EFFEKTE VON ALKYLGLUCOSIDEN UND NICHTIONISCHEN GEMINI-ZUCHERTENSIDEN", TENSIDE, SURFACTANTS, DETERGENTS, CARL HANSER VERLAG, MUNCHEN, DE, vol. 39, no. 2, 1 February 2002 (2002-02-01), pages 28-30, XP001112743, ISSN: 0932-3414
- ROSEN M J: "GEMINIS: A NEW GENERATION OF SURFACTANTS", CHEMTECH, WASHINGTON, DC, US, 1 March 1993 (1993-03-01), pages 30-33, XP000645619, ISSN: 0009-2703
- DATABASE GNPD [Online] MINTEL; 1 February 2015 (2015-02-01), "Facial Lotion", Database accession no. 2899363

## Description

The present invention concerns new solubilizing agents, in particular new agents for solubilizing fatty phase(s) in aqueous compositions.

The present invention also concerns new aqueous compositions comprising said solubilizing agents.

Conventionally, in the fields of cosmetics such as deodorants, bath agents, eau-de-toilettes, deodorizers or the like, various products exhibiting a liquid state at ordinary temperature, have been prepared by solubilizing fatty phase such as colorants, vitamins, anti-oxidants, fats, oils ... in an aqueous solvent by using alcohols and/or surfactant(s).

Several drawbacks result from the use of alcohols, such as their inflammability, the risks associated with their storage or their transportation.... There is thus a need to reduce their content in cosmetic compositions, or even to avoid their use.

Ethoxylated surfactants resulting from petrochemical synthesis are known as substitutes of alcohols. However, such surfactants lead to the formation of formaldehyde and 1,4-dioxane (by-products) which are toxic.

There is thus a need to replace petroleum-derived raw materials with renewable ones, especially from an environmental point of view. There is also a need to provide new solubilizing agents which avoid the above-mentioned drawbacks for the known solubilizing agents.

In the literature, other surfactants than ethoxylated surfactants are known. However, a high content of said surfactants is typically required to solubilize fatty phase in aqueous solvent, and they are less efficient than ethoxylated surfactants. Large amounts of surfactant usually result in skin irritation.... In the field of cosmetics, it is thus particularly interesting to avoid or reduce the amount of surfactants. For example, US 5,789,371 describes amphoteric gemini surfactants having the formula:

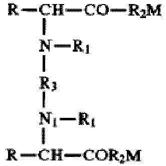

The transparent or translucent appearance of cosmetics is highly satisfactory from an esthetic viewpoint and can for this reason be of great commercial interest. A clear and transparent appearance of cosmetic products has advantages in the market since it can be attributed pureness, mildness, cleanliness, freshness or lightness to consumers. Another benefit of a clear appearance, in combination with a transparent packaging, is that the consumer is readily able to view and inspect the product.

There is thus a need for providing a solubilizing agent which may be effective at low concentrations for solubilizing fatty phase in aqueous compositions.

There is also a need for a solubilizing agent which allows solubilization of a similar or even higher amount of fatty phase in aqueous compositions, than conventional surfactants.

There is thus also a need for providing transparent aqueous compositions.

The aim of the present invention is to provide a solubilizing agent which derives from renewable raw materials.

Another aim of the present invention is to provide a solubilizing agent which avoids the formation of toxic by-products such as dioxane or formaldehyde.

Another aim of the present invention is to provide a solubilizing agent which is efficient for solubilizing fatty phase in aqueous compositions, in particular at low content.

Another aim of the present invention is to provide a solubilizing agent which allows the solubilization of a high amount of fatty phase in aqueous compositions.

Another aim of the present invention is to provide a solubilizing agent for preparing transparent aqueous compositions.

The present invention concerns a composition comprising:
a) at least one gemini surfactant having the formula (A) or (I-1) as defined below;
b) at least one alkylpolyglucoside; and
c) at least one polyglycerol fatty acid(s) ester, wherein the fatty acid comprises from 4 to 36 carbon atoms, wherein said composition comprises from 0.1% to 20% by weight of gemini surfactant(s) based on the total weight of the composition.

As used herein, said above-mentioned composition is named composition C1.

### Gemini surfactant

As used herein, the term "gemini surfactant" means a surfactant comprising at least two hydrophilic heads linked together via a spacer, and at least two hydrophobic tails, each hydrophobic tail being bonded to one hydrophilic head. The spacer may be polar or apolar, flexible or rigid, short or long. The hydrophilic heads can be anionic, cationic or non-ionic. In particular, the gemini surfactant comprises two hydrophilic heads linked together via a spacer, and two hydrophobic tails.

A number of the gemini surfactants are reported in the literature, see for example, Okahara et al., J. Japan Oil Chem. Soc. 746 (Yukagaku) (1989); Zhu et al., 67 JAOCS 7,459 (July 1990); Zhu et al., 68 JAOCS 7,539 (1991); Menger et al., J. Am. Chemical Soc. 113,1451 (1991); Masuyama et al. , 41 J. Japan Chem. Soc. 4,301 (1992); Zhu et al., 69 JAOCS 1,30 (January 1992); Zhu et al. , 69 JAOCS 7,626 July 1992); Menger et al., 115 J. Am. Chem. Soc. 2,10083 (1993); Rosen, Chemtech 30 (March 1993); and Gao et al., 71 JAOCS 7, 771 (July 1994).

The following gemini surfactants may also be describes: 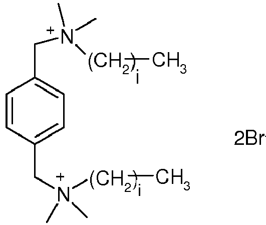 wherein i is an integer comprised from 1 to 12; 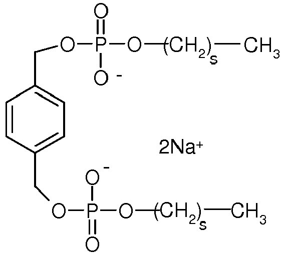 wherein s is an integer comprised from 1 to 12; 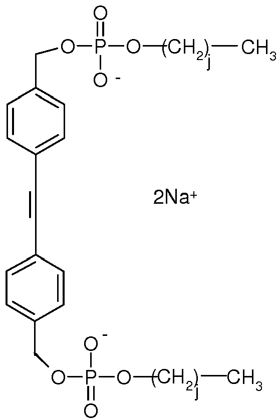 wherein j is an integer comprised from 1 to 12; 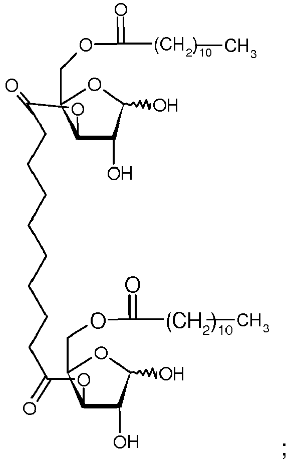 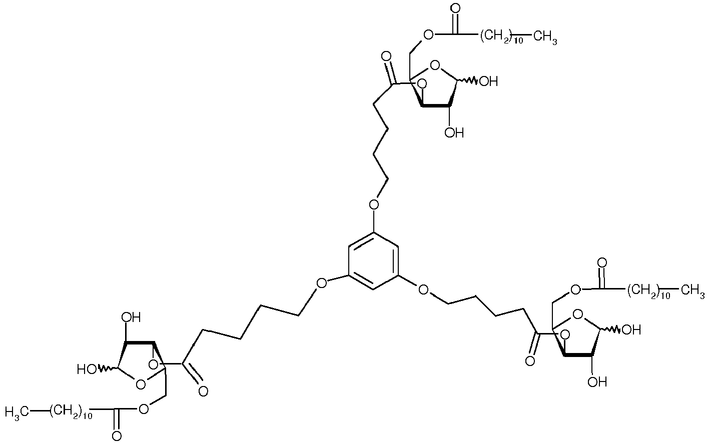 and their isomers.

According to the invention, the gemini surfactant has the following formula (A): 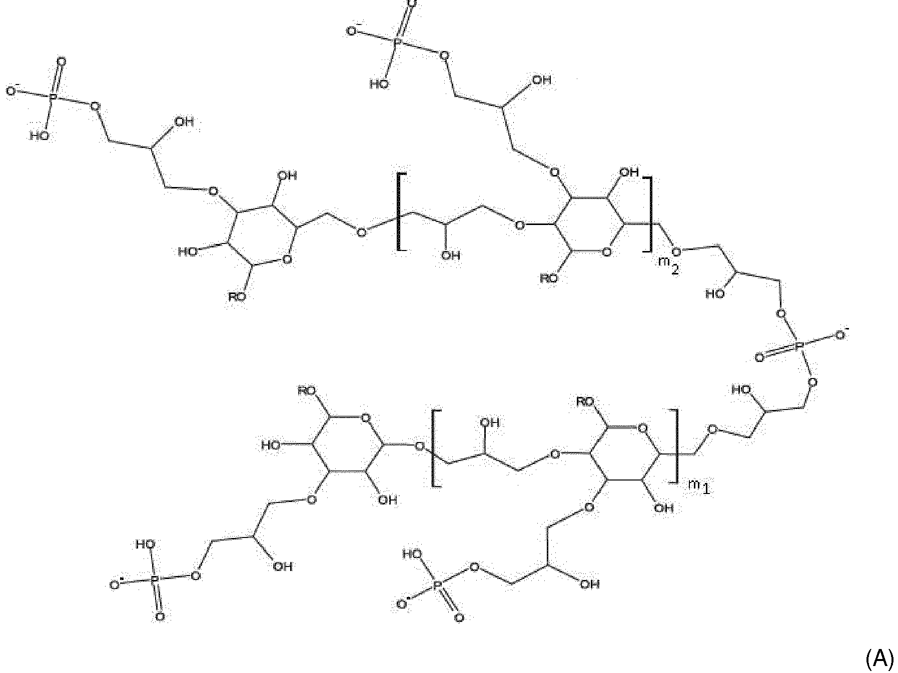 wherein R represents a linear or branched alkyl group comprising from 1 to 26 carbon atoms, preferably from 1 to 12 carbon atoms, m₁ and m2 are, independently of each other, integer comprised between 1 and 20, y being an integer wherein y = m₁ + m₂ + 3, Z being H⁺ or Na⁺.

In one embodiment, R is selected from the group consisting of: decyl, cocoyl, and lauryl. In particular R, is a lauryl group.

In one embodiment, Z is Na⁺.

In particular, the gemini surfactant is choosen from the group consisting of: poly(sodium decylglucoside hydroxypropylphosphate), poly(sodium laurylglucoside hydroxypropylphosphate), poly(sodium cocoylglucoside hydroxypropylphosphate and mixtures thereof).

In particular, the gemini surfactant used in the present invention is sodium hydroxypropylphosphate decylglucoside crosspolymer or sodium hydroxypropylphosphate laurylglucoside crosspolymer or sodium hydroxypropylphosphate cocoglucoside crosspolymer, in particular sold by colonial Chemical, Inc under the trade name Poly Suga® Phos.

The present application also describes gemini surfactants having the following formula (A'): 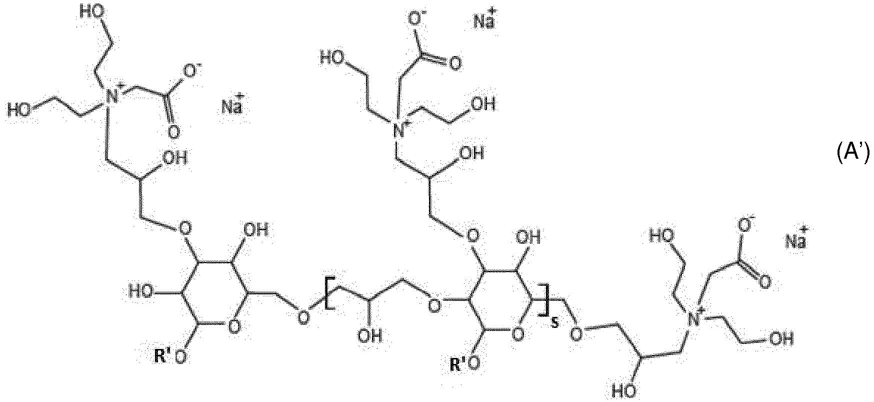 wherein s is an integer comprised from 1 to 20, preferably from 1 to 5, and R' represents a linear or branched alkyl group comprising from 1 to 26 carbon atoms, preferably from 1 to 18, and more preferably from 1 to 12, carbon atoms.

In one embodiment, R' is selected from the group consisting of: decyl, cocoyl, lauryl and mixtures thereof. In particular R' is a lauryl group. In particular, R' is cocoyl.

In particular, the gemini surfactant used in the present invention is selected from the group consisting of: hydroxypropylphosphate decylglucoside crosspolymer, sodium didecylglucoside hydroxypropylphosphate, sodium hydroxypropylphosphate cocoglucoside crosspolymer, sodium hydroxypropylphosphate laurylglucoside crosspolymer, poly(sodium dilaurylglucoside hydroxypropylphosphate), poly(sodium decylglucoside hydroxypropylphosphate), poly(sodium laurylglucoside hydroxypropylphosphate), poly(sodium cocoylglucoside hydroxypropylphosphate), and mixtures thereof.

The present application also describes gemini surfactants having the following formula (I): 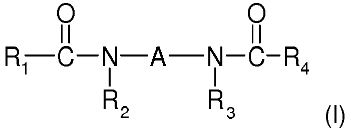 wherein:
- R₁ and R₄ represent, independently of each other, a linear or branched alkyl group comprising from 1 to 36 carbon atoms, preferably from 1 to 22, and more preferably from 6 to 12 carbon atoms, or an aryl group comprising from 6 to 24 carbon atoms;
- R₂ and R₃ represent, independently of each other, H or a linear or branched alkyl group comprising from 1 to 22 carbon atoms, preferably from 6 to 12 carbon atoms; and
- A represents a radical having the following formula (II): 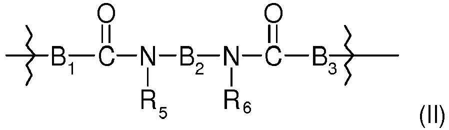 wherein:
   ∘ R₅ and R₆ represent, independently of each other, H or a linear or branched alkyl group comprising from 1 to 22 carbon atoms, preferably from 6 to 12 carbon atoms;
   ∘ B₁ represents a radical having the following formula (III): 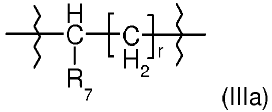 wherein
      R₇ represents the following radical: 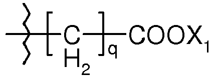
      X₁ being H or M, M being an alkali metal or alkaline-earth metal, and q being an integer comprised between 0 and 10;
   ∘ B2 represents a radical having the following formula (IIIb): 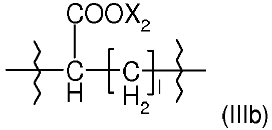 X2 being H or M, M being an alkali metal or alkaline-earth metal, and I being an integer comprised between 0 and 10; and
   ∘ B3 represents a radical having the following formula (IIIc): 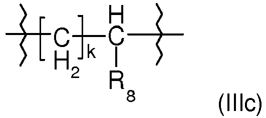 wherein:
      R₈ represents the following radical: 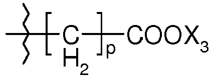
      X3 being H or M, M being an alkali metal or alkaline-earth metal, and p being an integer comprised between 0 and 10;
   or their diastereoisomers.

In formula (I) above-mentioned, R₁ and R₄ may represent, independently of each other, a linear or branched alkyl group comprising from 10 to 12 carbon atoms, and preferably 11 carbon atoms.

In formula (I), both R₂ and R₃ may represent H.

In formula (I), both R₅ and R₆ may represent H.

In formula (I), R₇ may represent COOX₁, X₁ being as defined above, in particular - COOH or -COONa.

In formula (I), R₇ may represent -(CH₂)₂-COOX₁, X₁ being as defined above.

In formula (I), R₈ may represent -COOX₃, X₃ being as defined above, in particular - COOH or -COONa.

In formula (I), R₈ may represent -(CH₂)₂-COOX₃, X3 being as defined above.

In formula (I), X₂ is H or Na.

In formula (I), p, l, q and r may, independently of each other, be comprised from 0 to 6, preferably from 0 to 2.

In formula (I), B2 may represent the following radical: 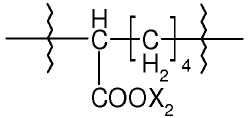 X2 being as defined above.

In formula (I), B₁ may represent one of the following radicals: 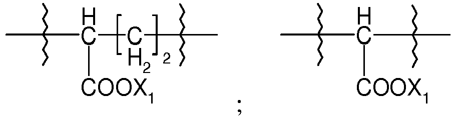 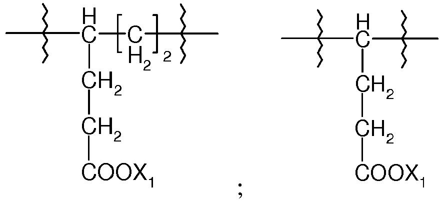 X₁ being as defined above.

In formula (I), B3 may represent of the following radicals: 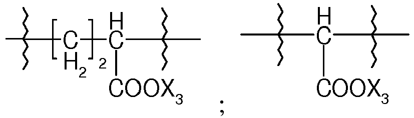 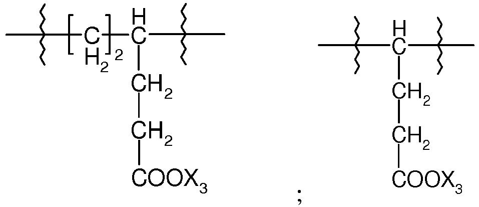 X3 being as defined above.

In one embodiment, the gemini surfactant has the following formula (I-1): 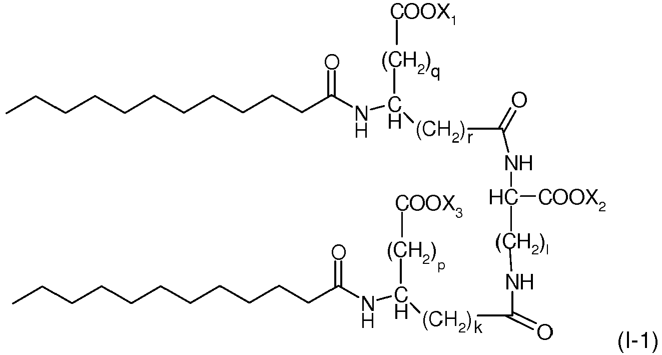 or their diastereoisomers, X₁, X₂, X₃, r, q, I, p and k being as defined above.

In one embodiment, in the above-mentioned formula, when q = 2, then r = 0.

In one embodiment, in the above-mentioned formula, when q = 0, then r = 2.

In one embodiment, in the above-mentioned formula, when k = 2, then p = 0.

In one embodiment, in the above-mentioned formula, when k = 0, then p = 2.

In one embodiment, the gemini surfactant has the following formula (I-2): 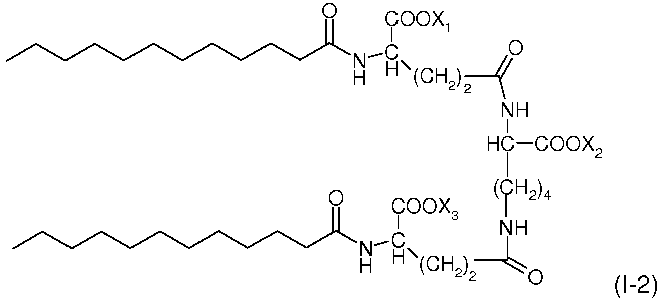 or their diastereoisomers, X₁, X₂ and X₃ being as defined above, in particular X₁, X₂ and X₃ being, independently of each other, H or Na.

In particular, the gemini surfactant used in the present invention is sodium dilauramidoglutamide lysine sold by Asahi Kasei Group under the trade name Pellicer L-30® or Pellicer LB-10®.

In particular, the gemini surfactant used in the present invention is selected from the group consisting of: sodium dilauramidoglutamide lysine, hydroxypropylphosphate decylglucosides crosspolymer, sodium didecylglucoside hydroxypropylphosphate, sodium hydroxypropylphosphate cocoglucoside crosspolymer, sodium hydroxypropylphosphate laurylglucoside crosspolymer, poly(sodium dilaurylglucoside hydroxypropylphosphate), poly(sodium decylglucoside hydroxypropylphosphate), poly(sodium laurylglucoside hydroxypropylphosphate), poly(sodium cocoylglucoside hydroxypropylphosphate), and mixtures thereof.

In particular, the gemini surfactant used in the present invention is selected from the group consisting of: sodium dilauramidoglutamide lysine, sodium didecylglucoside hydroxypropylphosphate, sodium hydroxypropylphosphate cocoglucoside crosspolymer, sodium hydroxypropylphosphate laurylglucoside crosspolymer, poly(sodium dilaurylglucoside hydroxypropylphosphate), and mixtures thereof.

According to the invention, the composition comprises from 0.1% to 20% by weight of gemini surfactant(s), based on the total weight of the composition. In one embodiment, the composition according to the invention comprises from 0.25% to 10%, and more preferably from 1% to 6% by weight of gemini surfactant(s), based on the total weight of the composition. In particular, the composition comprises from 1% to 5%, and more preferably from 1.45% to 3.0% by weight of gemini surfactant(s) based on the total weight of the composition.

The content of gemini surfactant(s) in the composition according to the invention corresponds to the total content of gemini surfactant(s) in said composition. For example, when the composition comprises two different gemini surfactants, the content of gemini surfactants in the composition corresponds to the sum of the content of each gemini surfactant in the composition.

As used herein, the percentages by weight are percentages by weight of active ingredient.

### Alkvlpolvalucosides (APG)

According to the invention, the composition comprises at least one alkylpolyglucoside.

In one embodiment, the composition comprises a mixture of alkylpolyglucosides.

Alkylpolyglucosides are widely used in the cosmetic field.

Typically, alkylpolyglucosides result from the condensation reaction of fatty alcohol(s) and of glucose or one of its derivatives.

In one embodiment, alkylpolyglucosides are prepared starting from a mixture of fatty alcohols with various alkyl chain lengths, in particular each alkyl chain comprising from 2 to 22 carbon atoms, preferably from 8 to 18 carbon atoms.

For example, the capryl/caprylyl glucoside results from the condensation reaction of a mixture of caprylic and decyl alcohols, with glucose.

In particular, alkylpolyglucosides are blends of alkylpolyglucosides having different alkyl chain lengths, and/or having various degree of polymerization, namely various glucose (or glucose derivative) units.

According to the invention, the term "alkylpolyglucoside" designates an alkylmonoglucoside (degree of polymerization 1) or an alkylpolyglucoside (degree of polymerization greater than 1).

According to the invention, the alkylpolyglycosides may be used alone or in the form of mixtures of several alkylpolyglycosides.

In one embodiment, the alkylpolyglucoside (APG) has the following formula (IV):

Rₐ(O)(G)ₓ (IV)

wherein:
- Rₐ represents a linear or branched alkyl group comprising from 2 to 22 carbon atoms;
- G represents a sugar moiety comprising 5 or 6 carbon atoms; and
- x is an integer comprised between 1 and 15, preferably between 1 and 10.

In one embodiment, preferred alkylpolyglucosides are those in which Rₐ consists:
- essentially of C8 and C10 alkyl groups;
- essentially of C12 and C14 alkyl groups;
- essentially of C8 to C16 alkyl groups; or
- essentially of C12 to C16 alkyl groups.

In one embodiment, in formula (IV) above-mentioned, G represents a glucose, a fructose or a galactose moiety. In particular, G represents a glucose moiety.

In one embodiment, Rₐ may be a mixture of linear or branched alkyl groups comprising from 2 to 22 carbon atoms. For example, the alkylpolyglucoside of formula (I) may be a polyglucoside wherein Rₐ is octyl and/or decyl.

In one embodiment, in formula (IV), Rₐ is a linear alkyl group comprising from 8 to 10 carbon atoms.

In one embodiment, in formula (IV), x is an integer comprised from 1 to 2, and preferably from 1.1 to 1.5.

In one embodiment, in formula (IV), Rₐ is a linear or branched alkyl group comprising from 8 to 18 carbon atoms, preferably from 8 to 10 carbon atoms, G is a glucose moiety and x is an integer comprised between 1 and 2.

In one embodiment, the alkylpolyglucoside (APG) has the following formula (IV'):

Rₐ(O)(G)ₓ (IV')

wherein Rₐ is a mixture of linear or branched alkyl groups comprising from 2 to 22 carbon atoms.

In one embodiment, the composition comprises a mixture of at least two different compounds having the above-mentioned formula (IV).

For example, the composition comprises a mixture of at least two different compounds having the above-mentioned formula (IV), which differ in the nature of Rₐ and/or the value of x.

In one embodiment, the composition comprises a mixture of:
- one compound of formula (IV) wherein Rₐ is a linear alkyl group comprising 8 carbon atoms; and
- one compound of formula (IV) wherein Rₐ is a linear alkyl group comprising 10 carbon atoms.

In one embodiment, the alkylpolyglucoside is selected from the group consisting of: cocoglucoside (INCI), decyl glucoside (INCI), lauryl glucoside (INCI), caprylyl/capryl glucoside (INCI), arachidyl glucoside (INCI), butyl glucoside (INCI), caprylyl glucoside (INCI), cetearyl glucoside (INCI), coco glucoside (INCI), ethyl glucoside (INCI), hexadecyl D-glucoside (INCI), isostearyl glucoside (INCI), lauryl glucoside (INCI), myristyl glucoside (INCI), octadecyl D-glucoside (INCI), octyldodecyl glucoside (INCI), undecyl glucoside (INCI), and their mixtures.

In one embodiment, the alkylpolyglucoside is selected from the group consisting of: (C12-C20)alkylpolyglucoside, (C8-C16)lalkylpolyglucoside, (C8-C10)alkylpolyglucoside, (C12-C16)alkylpolyglucoside, (C12-C14)alkylpolyglucoside, and their mixtures.

In particular, as known in the cosmetic field, the above-mentioned INCl names of the alkylpolyglucoside cover a blend of fatty alcohol glucosides having various alkyl chain length and/or degree of polymerization.

For example, the lauryl glucoside is a (C12-C16) alkylpolyglucoside.

Preferred APG are in particular chosen from cocoglucoside (INCI) commercialized under the trade name Plantacare 818® from BASF or FM 818® from FENCHEM BIOTEK LTD; decyl glucoside (INCI) commercialized under the trade name Plantacare 2000® UP from BASF or FC 10® from FENCHEM BIOTEK LTD or FM 2000® from FENCHEM BIOTEK LTD or RITAFACTANT LG® from RITA or ORAMIX NS10® from SEPPIC or MYDOL 10® from KAO; lauryl glucoside (INCI) commercialized under the trade name Plantacare 1200® from BASF or FM 600® from FENCHEM BIOTEK LTD or FM 1200® from FENCHEM BIOTEK LTD or MYDOL 12® from KAO; caprylyl/capryl glucoside (INCl) commercialized under the trade name Oramix CG 110® from Seppic or Plantacare KE 3711® from BASF or FM 8170® from FENCHEM BIOTEK LTD or HELIWET APG® from MOSSELMAN or APG 0810® from POLYMET or TRITON CG110® from DOW, and their mixtures.

In one embodiment, the composition according to the invention comprises from 20% to 70%, preferably from 20% to 50%, and more preferably from 25% to 45%, by weight of alkylpolyglycoside(s), based on the total weight of the composition.

The content of alkylpolyglucoside(s) in the composition according to the invention corresponds to the total content of alkylpolyglucoside(s) in said composition. For example, when the composition comprises two different alkylpolyglucosides, the content of alkylpolyglucosides in the composition corresponds to the sum of the content of each alkylpolyglucoside in the composition.

### Polyalycerol fatty acid ester

According to the invention, the above-mentioned composition comprises at least one polyglycerol fatty acid(s) ester.

In one embodiment, the composition according to the invention comprises two or three polyglycerol fatty acid(s) esters.

As used herein, the term "polyglycerol fatty acid(s) ester" means an ester of polyglycerol and of at least one fatty acid. For example, the polyglycerol fatty acids ester may be an ester of polyglycerol and of a mixture of two fatty acids of different nature.

As used herein, the term "polyglycerol" refers to polymers of glyceryl. Polyglycerol is typically a linear chain of 2 to 20, preferably 2 to 10 units of glycerol.

In one embodiment, the polyglycerol fatty acid(s) ester according to the invention results from the esterification of at least one linear or branched, saturated or unsaturated, fatty acid having from 4 to 36 carbon atoms, and a polyglycerol.

In one embodiment, the fatty acid is a linear or branched, saturated or unsaturated, carboxylic acid, comprising from 4 to 36 carbon atoms, preferably 4 to 22 carbon atoms, more preferably 6 to12 carbon atoms, in particular 7 to 10 carbon atoms.

In one embodiment, the fatty acid is selected from the group consisting of: caprylic acid, capric acid, oleic acid, lauric acid, myristic acid, ricinoleic acid, palmitic acid, stearic acid, arachidic acid, isostearic acid, behenic acid, and their mixtures.

Preferably, the fatty acid is a saturated monocarboxylic fatty acid comprising from 8 to 18 carbon atoms. In particular, the fatty acid is capric acid or lauric acid.

In one embodiment, the polyglycerol fatty acid(s) ester according to the invention is selected from the group consisting of: polyglyceryl-4 caprylate/caprate, polyglyceryl-5 caprylate/caprate, polyglyceryl-6 caprylate/caprate, polyglyceryl-7 caprylate/caprate, polyglyceryl-8 caprylate/caprate, polyglyceryl-9 caprylate/caprate, polyglyceryl-10 caprylate/caprate, polyglyceryl-3 caprate, polyglyceryl-4 caprate, polyglyceryl-5 caprate, polyglyceryl-6 caprate, polyglyceryl-7 caprate, polyglyceryl-8 caprate, polyglyceryl-9 caprate, polyglyceryl-10 caprate, polyglyceryl-10 decastearate, polyglyceryl-3 diisostearate, polyglyceryl-10 diisostearate, polyglyceryl-4 laurate, polyglyceryl-5 laurate, polyglyceryl-6 laurate, polyglyceryl-7 laurate, polyglyceryl-8 laurate, polyglyceryl-9 laurate, polyglyceryl-10 laurate, polyglyceryl-3 cocoate, polyglyceryl-4 cocoate, polyglyceryl-6 cocoate, polyglyceryl-7 cocoate, polyglyceryl-8 cocoate, polyglyceryl-9 cocoate, polyglyceryl-10 cocoate, polyglyceryl-11 cocoate, polyglyceryl-12 cocoate, polyglyceryl 10-isostearate, polyglyceryl-6 myristate, polyglyceryl-7 myristate, polyglyceryl-8 myristate, polyglyceryl-9 myristate, polyglyceryl-10 myristate, polyglyceryl-11 myristate, polyglyceryl-12 myristate, polyglyceryl-10 oleate, polyglyceryl-11 oleate, polyglyceryl-12 oleate, polyglyceryl-3 palmitate, polyglyceryl-10 palmitate, polyglyceryl-3 polyricinoleate, polyglyceryl-6 polyricinoleate, polyglyceryl-10 polyricinoleate polyglyceryl-3 stearate, polyglyceryl-10 stearate, polyglyceryl-11 stearate, polyglyceryl-12 stearate, and mixtures thereof.

In particular, the polyglycerol fatty acid(s) ester is polyglyceryl-6 laurate or polyglyceryl-4 caprate.

In particular, the polyglycerol fatty acid(s) ester is selected from the group consisting of polyglyceryl-6 laurate, polyglyceryl-10-isostearate, polyglyceryl-4 caprate, and mixtures thereof.

In particular, the polyglycerol fatty acid(s) ester is polyglyceryl-4 caprate commercialized under the trade name MASSOCARE PG4-C from MASSO or HYDRIOL PGCL.4 from HYDRIOL or TEGOSOFT PC 41 from EVONIK INDUSTRIES or polyglycerol-4 caprate from MOSSELMAN.

In particular, the polyglycerol fatty acid(s) ester is polyglyceryl-6 laurate commercialized under the trade name HEXAGLYN 1-L from NIKKOL or S-FACE L-601 from SAKAMOTO.

In particular, the polyglycerol fatty acid(s) ester is polyglyceryl-10 isostearate commercialized under the trade name Decaglyn 1-ISV from NIKKOL or S-FACE IS-1001P from SAKAMOTO
In one embodiment, the composition according to the invention comprises a mixture of polyglyceryl-6 laurate and polyglyceryl-4 caprate.

In one embodiment, the composition according to the invention comprises from 1% to 60%, preferably from 2% to 50%, and more preferably from 5% to 40% by weight of polyglycerol fatty acid(s) ester(s), based on the total weight of the composition. In particular, the composition comprises from 15% to 40%, preferably from 20% to 30%, and more preferably from 25% to 30% by weight of polyglycerol fatty acid(s) ester(s), based on the total weight of the composition.

The content of polyglycerol fatty acid(s) ester(s) in the composition according to the invention corresponds to the total content of polyglycerol fatty acid(s) ester(s) in said composition. For example, when the composition comprises two different polyglycerol fatty acid(s) esters, the content of polyglycerol fatty acid(s) esters in the composition corresponds to the sum of the content of each polyglycerol fatty acid(s) ester in the composition.

### Sugar fatty acid(s) ester

In one embodiment, the composition of the invention further comprises at least one sugar fatty acid(s) ester.

As used herein, the term "sugar fatty acid(s) ester" means an ester of sugar and of at least one fatty acid. For example, a sugar fatty acids ester is an ester of sugar and of a mixture of different fatty acids.

In one embodiment, sugar fatty acid esters result from the esterification of at least one fatty acid (or an alkyl ester of said fatty acid), with sugar. For example, the sucrose fatty acid(s) ester results from the esterification of sucrose with at least one fatty acid, such as lauric acid, stearic acid, myristic acid, or oleic acid.

In particular, an alkyl ester of a fatty acid is a methyl or an ethyl ester of fatty acid. For example, an alkyl ester of stearic acid is methyl stearate or ethyl stearate.

As used herein, the term "sugar" means a carbohydrate selected from oses and osides, and comprising from 1 to 10 monosaccharide units. For example, mention may be made to threose, erythrose, desoxyribose, ribose, xylose, ribulose, lyxose, glucose, fructose, idose, sorbose, galactose, allose, maltose, lactose, isomaltose, sucrose, isomaltulose, cellobiose, and saccharose, raffinose, melezitose. Osides include a linear or branched alkyl glycoside chain comprising from 1 to 12 carbon atoms.

In one embodiment, the sugar is selected from the group consisting of: glucose, fructose, galactose, sucrose, maltose, lactose, and their mixtures. In particular, the sugar is sucrose.

In one embodiment, the sugar fatty acid(s) esters are sucrose fatty acid(s) esters.

According to the invention, the sugar fatty acid(s) ester may be blends of different esters, such as sugar fatty acid monoesters, diesters, triesters, and polyesters. The sucrose fatty acid(s) ester typically contains sucrose fatty acid(s) monoesters. The sucrose fatty acid(s) esters containing monoesters may further contain one or more of sucrose fatty acid(s) diesters, triesters, and/or polyesters. In one example, the sucrose fatty acid(s) ester contains one or more of sucrose stearate, sucrose laurate, sucrose palmitate, sucrose oleate, sucrose caprylate, sucrose decanoate, sucrose myristate, sucrose pelargonate, sucrose undecanoate, sucrose tridecanoate, sucrose pentadeconoate, sucrose heptadecanoate, and homologs thereof, including mono-, di-, tri, and polyester forms of these sucrose fatty acid(s) esters.

The sucrose fatty acid(s) ester may include blends of sucrose fatty acid(s) esters, containing a plurality of different sucrose fatty acid(s) esters. The different sucrose fatty acid(s) esters in the blend may vary in the length and/or saturation of the carbon chain of the fatty acid portion of the ester, or in the degree of esterification (e.g., whether the ester is a monoester, diester, triester, or polyester). Typically, the sucrose fatty acid(s) ester contains proportionally more monoesters than other types of esters (e.g., diesters, triesters, and polyesters).

In one embodiment, the fatty acid is a linear or branched, saturated or unsaturated, carboxylic acid, comprising from 4 to 36 carbon atoms, preferably 4 to 22 carbon atoms, more preferably 6 to 12 carbon atoms.

In one embodiment, the fatty acid is selected from the group consisting of: erucic acid, caprylic acid, capric acid, oleic acid, caproic acid, lauric acid, myristic acid, palmitic acid, stearic acid, arachidic acid, isostearic acid, behenic acid, and their mixtures. In particular, the fatty acid is selected from the group consisting of: lauric acid, myristic acid, oleic acid, palmitic acid and stearic acid.

Preferably, the fatty acid is a saturated monocarboxylic fatty acid comprising from 8 to 18 carbon atoms. In particular, the fatty acid is capric acid or lauric acid.

In one embodiment, the sugar fatty acid(s) ester is selected from the group consisting of: sucrose erucate, sucrose behenate sucrose laurate, sucrose myristate, sucrose oleate, sucrose palmitate, sucrose stearate, and mixtures thereof. In particular, the sugar fatty acid(s) ester is sucrose laurate.

In particular, the sugar fatty acid(s) ester is sucrose behenate commercialized under the trade name of RYOTO SUGAR ESTER B-370 from MITUBISHI-KAGAKU FOODS or Cosmelike B-30 from DAI-ICHI KOGYO SEIYAKU.

In particular, the sugar fatty acid(s) ester is sucrose erucate is commercialized under the trade name of RYOTO SUGAR ESTER ER-190 from MITUBISHI-KAGAKU FOODS or RYOTO SUGAR ESTER ER-290 from MITUBISHI-KAGAKU FOODS or COSMELIKE R-10 from DAI-ICHI KOGYO SEIYAKU or COSMELIKE R-20 from DAI-ICHI KOGYO SEIYAKU.

In particular, the sugar fatty acid(s) ester is sucrose laurate commercialized under the trade name of RYOTO SUGAR ESTER L-195 from MITUBISHIKAGAKU FOODS or RYOTO SUGAR ESTER L-595 from MITUBISHI-KAGAKU FOODS or RYOTO SUGAR ESTER LWA-1570 from MITUBISHI-KAGAKU FOODS or RYOTO SUGAR ESTER L-1695 from MITUBISHI-KAGAKU FOODS or COSMELIKE L-10 from DAI-ICHI KOGYO SEIYAKU or COSMELIKE L-50 from DAI-ICHI KOGYO SEIYAKU or COSMELIKE L-150A from DAI-ICHI KOGYO SEIYAKU or COSMELIKE L-160A from DAI-ICHI KOGYO SEIYAKU or COSMELIKE L-160 from DAI-ICHI KOGYO SEIYAKU or SURFHOPE C-1215 L from MITUBISHIKAGAKU FOODS or SURFHOPE C-1216 from MITUBISHIKAGAKU FOODS.

In particular, the sugar fatty acid(s) ester is sucrose myristate commercialized under the trade name of COSMELIKE M-160 from DAI-ICHI KOGYO SEIYAKU.

In particular, the sugar fatty acid(s) ester is sucrose oleate commercialized under the trade name of RYOTO SUGAR ESTER O-170 from MITUBISHI-KAGAKU FOODS or RYOTO SUGAR ESTER OWA-1570 from MITUBISHI-KAGAKU FOODS or COSMELIKE O-10 from DAI-ICHI KOGYO SEIYAKU or COSMELIKE O-150 from DAI-ICHI KOGYO SEIYAKU.

In particular, the sugar fatty acid(s) ester is sucrose palmitate commercialized under the trade name of RYOTO SUGAR ESTER P-170 from MITUBISHI-KAGAKU FOODS or RYOTO SUGAR ESTER P-1570 from MITUBISHI-KAGAKU FOODS or RYOTO SUGAR ESTER P-1670 from MITUBISHIKAGAKU FOODS or COSMELIKE P-10 from DAI-ICHI KOGYO SEIYAKU or COSMELIKE P-160 from DAI-ICHI KOGYO SEIYAKU.

In particular, the sugar fatty acid(s) ester is sucrose stearate commercialized under the trade name of RYOTO SUGAR ESTER S-070 from MITUBISHIKAGAKU FOODS or RYOTO SUGAR ESTER S-170 from MITUBISHI-KAGAKU FOODS or RYOTO SUGAR ESTER S-270 from MITUBISHI-KAGAKU FOODS or RYOTO SUGAR ESTER S-370 from MITUBISHI-KAGAKU FOODS or RYOTO SUGAR ESTER S-370F from MITUBISHI-KAGAKU FOODS or RYOTO SUGAR ESTER S-570 from MITUBISHI-KAGAKU FOODS or RYOTO SUGAR ESTER S-770 from MITUBISHI-KAGAKU FOODS or RYOTO SUGAR ESTER S-970 from MITUBISHI-KAGAKU FOODS or RYOTO SUGAR ESTER S-1170 from MITUBISHI-KAGAKU FOODS or RYOTO SUGAR ESTER S-1570 from MITUBISHI-KAGAKU FOODS or RYOTO SUGAR ESTER S-1670 from MITUBISHI-KAGAKU FOODS or COSMELIKE S-10 from DAI-ICHI KOGYO SEIYAKU or COSMELIKE S-50 from DAI-ICHI KOGYO SEIYAKU or COSMELIKE S-70 from DAI-ICHI KOGYO SEIYAKU or COSMELIKE S-110 from DAI-ICHI KOGYO SEIYAKU or COSMELIKE S-160 from DAI-ICHI KOGYO SEIYAKU or COSMELIKE S-190 from DAI-ICHI KOGYO SEIYAKU or COSMELIKE SA-10 from DAI-ICHI KOGYO SEIYAKU.

In one embodiment, the composition according to the invention comprises from 0% to 10%, preferably from 0% to 8%, and more preferably from 2% to 5% by weight of sugar fatty acid(s) ester(s), based on the total weight of the composition.

The content of sugar fatty acid(s) ester(s) in the composition according to the invention corresponds to the total content of sugar fatty acid(s) ester(s) in said composition. For example, when the composition comprises two different sugar fatty acid(s) esters, the content of sugar fatty acid(s) esters in the composition corresponds to the sum of the content of each sugar fatty acid(s) ester in the composition.

### Other ingredients

In one embodiment, the composition according to the invention further comprises pentylene glycol, 1,2-hexanediol, 1,2-octanediol and/or isopentyldiol.

In one embodiment, the composition comprises from 0% to 10%, preferably from 0% to 8%, and more preferably 5% by weight of pentylene glycol, 1,2-hexanediol and/or 1,2-octanediol, based on the total weight of the composition.

The use of pentylene glycol advantageously allows avoiding germs in said composition.

In one embodiment, the composition according to the invention further comprises an aqueous phase, such as water.

In one embodiment, the composition according to the invention comprises from 0% to 50%, preferably from 0% to 40%, and more preferably from 1% to 20% by weight of water, based on the total weight of the composition.

In one embodiment, the composition according to the invention comprises:
a) from 0.5% to 10% by weight of gemini surfactant(s), in particular having a HLB of 8;
b) from 25% to 50% by weight of alkylpolyglucoside(s), in particular having a HLB of 16;
c) from 2% to 50% by weight of polyglycerol fatty acid(s) ester(s), in particular having a HLB of 14;
d) from 0% to 8% by weight of sugar fatty acid(s) ester(s), in particular having a HLB of 16; and
e) from 0% to 10% by weight of pentylene glycol;
based on the total weight of the composition.

In one embodiment, the composition according to the invention comprises:
a) from 0.5% to 10% by weight of sodium dilauramidoglutamide lysine or sodium hydroxypropylphosphate laurylglucoside crosspolymer;
b) from 25% to 50 % by weight of caprylyl/capryl glucoside;
c) from 2% to 50% by weight of a mixture of polyglyceryl-4-caprate and polyglyceryl-6-laurate;
d) from 0% to 8% by weight of sucrose laurate; and
e) 5% by weight of pentylene glycol;
based on the total weight of the composition.

In one embodiment, the composition according to the invention comprises:
a) from 0.5% to 10% by weight of sodium dilauramidoglutamide lysine, or sodium hydroxypropylphosphate laurylglucoside crosspolymer;
b) from 25% to 70 %, preferably 25 to 50% by weight of caprylyl/capryl glucoside;
c) from 2% to 50% by weight of polyglyceryl-4-caprate, polyglyceryl-6-laurate, polyglyceryl-10 isostearate or their mixtures;
d) from 0% to 8% by weight of sucrose laurate; and
e) from 0 to 5% by weight of pentylene glycol;
based on the total weight of the composition.

In one embodiment, the composition according to the invention is free of ethoxylated surfactants. In particular, the composition is PEG-free.

In one embodiment, the composition according to the invention is alcohol-free.

In one embodiment, the composition C1 has a HLB comprised from 13 to 17, preferably from 13.5 to 16.5, and more preferably from 14.0 to 16.2.

As used herein, the term "HLB" refers to the "hydrophilic-lipophilic balance" of a molecule, such as a surfactant. The HLB number increases with increasing hydrophilicity. The HLB system is a semi-empirical method to predict what type of surfactant properties a molecular structure will provide. The HLB system is based on the concept that some molecules have hydrophilic groups, other molecules have lipophilic groups, and some have both. The HLB of a surfactant can be calculated according to Griffin WC: "Classification of Surface- Active Agents by 'HLB,'" Journal of the Society of Cosmetic Chemists 1 (1949): 311; and Griffin WC: "Calculation of HLB Values of Non-Ionic Surfactants," Journal of the Society of Cosmetic Chemists 5 (1954): 259.

The HLB of a mixture of surfactants may be calculated by multiplying the proportion of each surfactant in the mixture by its HLB value and adding up the resulting values, as is known in the art.

### Preparation of composition C1

The present invention also concerns the process of preparation of the composition C1 as mentioned-above, comprising mixing together at ambient temperature all the ingredients a), b) and c), and optional ingredients as mentioned, until a transparent composition is obtained.

In one embodiment, when the composition C1 comprises sugar fatty acid(s) ester(s), the mixture is heated at 50°C until a transparent composition is obtained.

### Use

The present invention also concerns the use of the above-mentioned composition (composition C1) as solubilizing agent.

As used herein, the term "solubilizing agent" or "solubilizer" means an agent which allows the solubilization of a liquid or solid fatty phase in an aqueous media, in particular in an aqueous composition.

The inventor found that the solubilizing agent (composition C1) advantageously allows the obtaining of transparent aqueous compositions by solubilizing a fatty phase in an aqueous media. This is particularly interesting given that transparent cosmetic compositions are particularly suitable for the customers.

The solubilizing agent according to the invention is advantageously efficient at low concentrations for solubilizing fatty phase in aqueous compositions. This is particularly interesting in view of reducing, or even avoiding the impact of surfactants on skin, such as skin irritations...

The solubilizing agent according to the invention advantageously allows the solubilization of high amount of fatty phase in aqueous compositions.

The composition C1 according to the invention advantageously does not involve the formation of formaldehyde or 1-4-dioxane which are toxic. Besides, the composition C1 is advantageously based on renewable ingredients.

As used herein, the term « transparent composition » means a composition which is transparent with the naked eye.

### Cosmetic compositions

The present invention also concerns an aqueous composition C2 comprising:
- an aqueous phase;
- the composition C1 as defined above; and
- a fatty phase.

In one embodiment, the fatty phase comprises at least one compound selected from the group consisting of: coloring agents, odorous substances of synthetic origin, fragrance aromatic compounds, fatty acids, fatty acid esters, fatty alcohols, vitamins, natural extracts, oils, perfumes, UV filters and their mixtures.

In one embodiment, the fatty alcohols, saturated or unsaturated, linear or branched, comprise from 8 to 26 carbon atoms, are in particular selected from the group consisting of cetyl alcohol, stearyl alcohol, octyldodecanol, 2-butyloctanol, 2-hexyldecanol, 2-undecylpentadecanol, oleyl alcohol, linoleyl alcohol, pentaerythrytol, lauryl alcohol, benzyl alcohol, isostearyl alcohol, behenyl alcohol, cetearyl alcohol, isocetyl alcohol, myristyl alcohol, C₃₀-₅₀ alcohols, lanolin alcohol and their mixtures.

In one embodiment, the fatty acid esters are esters of fatty acids and fatty alcohols. In particular, the fatty acid is a linear or branched, saturated or unsaturated, carboxylic acid, comprising from 4 to 36 carbon atoms, preferably 4 to 22 carbon atoms, more preferably 6 to12 carbon atoms, in particular 7 to 10 carbon atoms.

In one embodiment, the fatty acid is selected from the group consisting of: caprylic acid, capric acid, lauric acid, myristic acid, palmitic acid, stearic acid, arachidic acid, behenic acid, lignoceric acid, cerotic acid and their mixtures.

In one embodiment, the fatty acid esters are methyl cocoate/ricinoleate/isostearate, cetearyloctanoate, pentaerythrylisostearate or benzyl nezoate.

In one embodiment, the odorous substances of synthetic origin are compounds comprising at least one of group selected from ester, ether, aldehyde, ketone, aromatic alcohol and hydrocarbon groups.

For example, as esters, mention may be made of benzyl acetate, benzyl benzoate, phenoxyethyl isobutyrate, p-tert-butylcyclohexyl acetate, citronellyl acetate, citronellyl formate, geranyl acetate, linalyl acetate, dimethylbenzylcarbonyl acetate, phenylethyl acetate, linalyl benzoate, benzyl formate, ethylmethylphenyl glycinate, alkylcyclohexyl propionate, styralyl propionate, benzyl salicylate, ethyl acetate, ethyl benzoate, ethyl butyrate, ethyl hexanoate, ethyl cinnamate, ethyl formate, ethyl heptanoate, ethyl isovalerate, ethyl lactate, ethyl nonanoate, ethyl pentanoate, geranyl acetate, geranyl butyrate, geranyl pentanoate, isobutyl acetate, isobutyl format, isoamyl acetate, isopropyl acetate, linalyl acetate, linalyl butyrate, linalylformate, methyl acetate, methyl anthranilate, methyl benzoate, methyl butyrate, methyl cinnamate, methylpentanoate, methyl phenylacetate, methyl salicylate, nonyl caprylate, octyl acetate, octyl butyrate, amyl acetate, pentyl butyrate, pentyl hexanoate, pentyl pentanoate, propyl acetate, propyl hexanoate, propyl isobutyrate and terpenyl butyrate.

For example, as ethers, mention may be made of benzyl ethyl ether.

For example, as aldehydes, mention may be made of linear alkanals comprising from 8 to 18 carbon atoms, citral, citronellal, citronellyloxyacetaldehyde, cyclamen aldehyde, hydroxycitronellal, lilial, and bourgeonal.

For example, as ketones, mention may be made of ionones, for example α-isomethylionone, and methyl cedryl ketone.

For example, as aromatic alcohols, mention may be made of terpene alcohols, such as anethole, citronellol, eugenol, isoeugenol, geraniol, linalol, phenylethyl alcohol, and terpineol.

In one embodiment, the oil is selected from the group consisting of: mineral oils, synthetic oils, essential oils, vegetable oils and their mixtures.

In one embodiment, the essential oils are selected from the group consisting of: aniseed oil; basil oil; bergamot oil; bitter almond oil; camphor oil; citrus oil; lemon oil; eucalyptus oil; geranium oil; grapefruit oil; ginger oil; camomile oil; spearmint oil, caraway oil, lime oil; mandarin oil; clove (blossom) oil, orange oil; peppermint oil; rose oil; rosemary oil; sage oil; yarrow oil; star aniseed oil; thyme oil; vanilla extract; juniper berry oil; wintergreen oil; cinnamon leaf oil; cinnamon bark oil; lavandin oil, and mixtures thereof.

In one embodiment, the vegetable oils are selected from the group consisting of: almond oil, apricot kernel oil, jojoba oil, argan oil, shear butter, grape seed oil, avocado oil, macadamia oil, sunflower oil, and mixture thereof.

In one embodiment, the mineral oils are selected from the group consisting of isohexadecane, cera microcristallina, ceresin, hydrogenated polyisobutene, isododecane, ozokerite, paraffinum liquidum, alkanes, paraffin, petrolatum, and mixtures thereof.

In one embodiment, the synthetic oils are selected from the group consisting of silicones and synthetic wax.

In one embodiment, the coloring agents are selected from the group consisting of: lycopene, paprika, anthocyanin, chlorophyll, curcuma, beta caratone, annatto, natural and synthetic pigments and mixtures thereof.

As used herein, the term "coloring agent" means an agent providing a color to the composition in which said agent is incorporated.

In one embodiment, fragrance aromatic compounds or perfumes are natural and/or synthetic mixtures of essential oils and odorous substances of synthetic origin.

In one embodiment, the aqueous composition C2 as defined above comprises from 0.5% to 30%, preferably from 1% to 25%, and more preferably from 1.2% to 22% by weight of the composition C1 as defined above, based on the total weight of said aqueous composition C2.

In one embodiment, the aqueous composition C2 comprises from 0.01% to 40%, preferably from 0.05% to 20%, and more preferably from 1% to 5% by weight of the fatty phase based on the total weight of said aqueous composition C2.

The aqueous composition C2 of the present invention typically comprises water in amounts ranging from about 50% to 99%, preferably from about 60% to about 98%, and more preferably from about 70% to about 97% by weight based on the total weight of the composition.

In one embodiment, the composition C2 is an emulsion.

In one embodiment, the aqueous composition C2 is a cosmetic composition.

In one embodiment, the aqueous composition C2 is selected from the group consisting of: a perfume, eau de toilette, a clear deodorant, a moisturizing lotion, a moisturizing spray, a micellar water, a cleansing lotion, a cleansing solution gel, a two phase cleansing lotion, a cleansing scrub gel, a remover such as a rinse off remover or leave on remover, an after shave alcohol free, a purifying water with essential oils, a D phase system, a natural sprayable hair conditioner, a wet wipe solution, a natural body splash, a styling gel, an antidandruff lotion, UV sunscreen, a bath preparation, a hair care preparation, a make-up preparation skin care products, sun care products, hair care products, make-up products, and skin care products.

In one embodiment, the aqueous composition C2 may also further comprise any additive usually used in the relevant field, for example selected from gums, resins, dispersants, semi-crystalline polymers, antioxidants, preservatives, neutralizers, antiseptic agents, UV protective agents, cosmetic actives, such as vitamins, moisturizing agents, emollients or collagen protective agents, and mixtures thereof.

Adjustment of the nature and of the amount of the additives present in the compositions according to the invention falls under the routine operations of one skilled in the art, so that the cosmetic properties and the desired stability properties of the latter are not affected.

In the whole description above, unless mentioned otherwise, the term of « comprised between x and y » or « ranging from x to y » corresponds to an inclusive range, i.e., the values x and y are included in the range.

### EXAMPLES

### Suppliers:

Sucrose Laurate: Ryoto
Caprylyl/Capryl Glucoside: BASF
Lauryl glucoside: BASF
Hexyl glucoside: Fenchem
Cocoyl glucoside: NASF
Polyglyceryl-4 Caprate: Mosselman
Polyglyceryl-6 Laurate: Nikkol chemical
Polyglyceryl-10 isostearate : Nikkol chemical
Sodium dilauramidoglutamide lysine(29%) and water (71%): Asahi Kasei Group
Sodium hydroxypropylphosphate laurylglucoside crosspolymer: Colonial Chemical Inc
Sodium bishydroxyethylglycinate cocoglucosides crosspolymer: Colonial Chemical Inc
Sodium bishydroxyethylglycinate laurylglucosides crosspolymer: Colonial Chemical Inc
Pentylene Glycol: Cosphatec
Woody fragrance : Givaudan
Musky fragrance : Givaudan
Essence pin Sibérie: Givaudan
Essence Eucalyptus globulus chine : Givaudan
Aldehyde Cylamen extra: Givaudan
Licopene: Lycored
Paprika: Yunnan rainbow Bio-tech

### A. Preparation of the compositions C1

### Compositions with sucrose laurate

The compositions were prepared as follows:
- Adding the gemini surfactant in a beaker of 250 mL at 50°C; then
- Adding the APG surfactant and mixing at 50°C; then
- Adding the polyglycerol fatty acid(s) ester(s) into the mixture at 50°C; then
- Adding the sugar fatty acid ester(s); and
- Mixing at 50°C until a transparent composition is obtained.

### Compositions without sucrose laurate

The compositions were prepared as follows:
- Adding the gemini surfactant in a beaker of 250 mL at ambient temperature; then
- Adding the APG surfactant and mixing; then
- Adding the polyglycerol fatty acid(s) ester(s) into the mixture; then
- Mixing at ambient temperature until a transparent composition is obtained.

The compositions A1 to A14 were prepared and are disclosed in the following table 1:

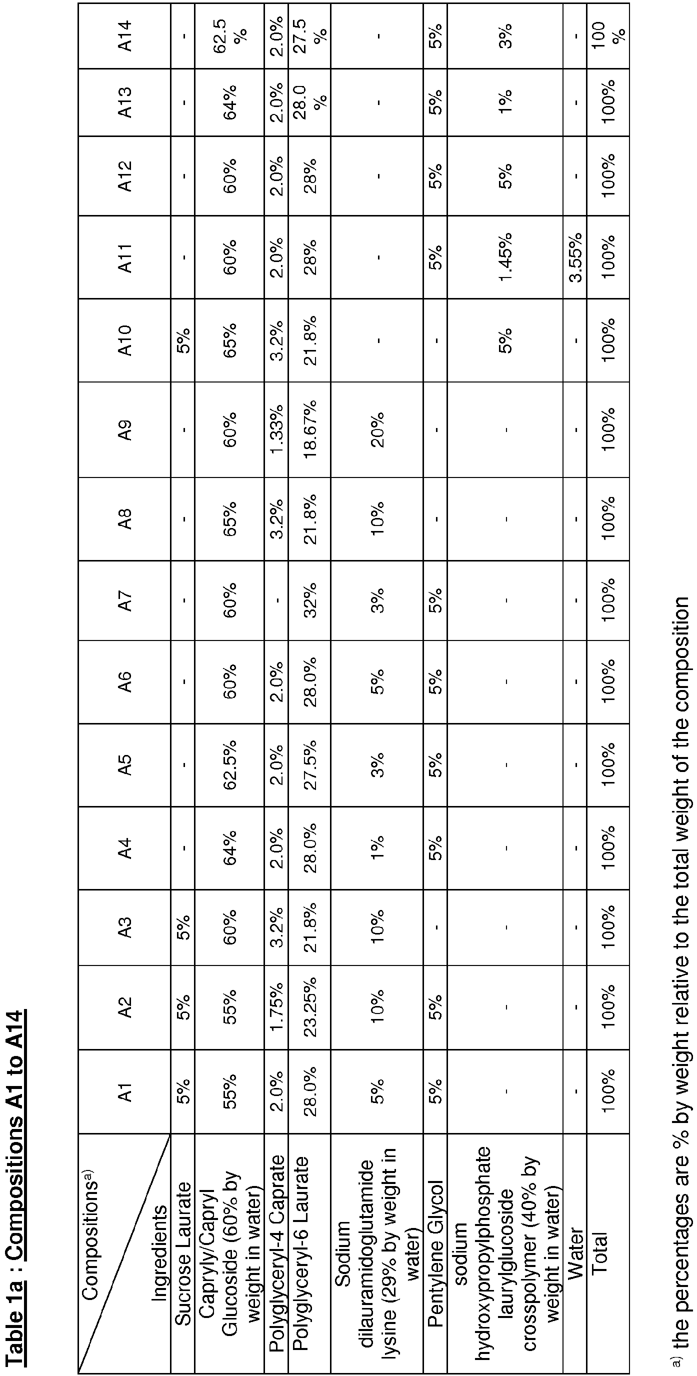

**Table 1b: Compositions A15 to A20**

| | | X1 (invention) | X2 | X3 | X4 (invention) | X5 | X6 |
|---|---|---|---|---|---|---|---|
| APG | Caprylyl/Capryl Glucoside (60% by weight in water) | 70% | 70% | 70% | 70% | 70% | 70% |
| Polyglycerol fatty acid ester | Polyglyceryl-4 Caprate | 25% | 25% | 25% | - | - | - |
| | Polyglyceryl-6 Laurate | - | - | - | 25% | 25% | 25% |
| gemini | Sodium dilauramidoglutamide lysine (29% by weight in water) | 5% | - | - | 5% | - | - |
| | Sodium bishydroxyethylglycinate Coco-glucosides crosspolymer (40% by weight in water) | - | 5% | - | - | 5% | - |
| | Sodium bishydroxyethylglycinate lauryl-glucosides crosspolymer (40% by weight in water) | - | - | 5% | - | - | 5% |
| | total | 100% | 100% | 100% | 100% | 100% | 100% |

### B. Preparation of the aqueous compositions

The following aqueous compositions were prepared by mixing a fatty phase with a composition selected from A1 to A20 (solubilizer prepared in section A), at ambient temperature, until a clear and transparent mixture is obtained. Then, the water was added slowly at the beginning, then in larger quantity to obtain the compositions B. The percentages are % by weight relative to the total weight of the compositions.

| Compositions | B 1.1 | B 1.2 | B 1.3 | B 1.4 | B 1.5 | B 1.6 | B 1.7 | B 1.8 |
|---|---|---|---|---|---|---|---|---|
| Solubilizer A1 | 5% | 12% | 22% | 4% | 3% | 15% | 9% | 14% |
| Fatty phase | woody 0.5% | woody 5% | Musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 2.1 | B 2.2 | B 2.3 | B 2.4 | B 2.5 | B 2.6 | B 2.7 | B 2.8 |
|---|---|---|---|---|---|---|---|---|
| Solubilizer A2 | 2% | 13% | 23% | 5% | 3% | 15% | 12% | 16% |
| fatty phase | woody 0.5% | woody 5% | musky 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 3.1 | B 3.2 | B 3.3 | B 3.4 | B 3.5 | B 3.6 | B 3.7 | B 3.8 |
|---|---|---|---|---|---|---|---|---|
| Solubilizer A3 | 2% | 12% | 22% | 4% | 3% | 15% | 12% | 16% |
| fatty phase | woody 0.5% | woody 5% | musky 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 4.1 | B 4.2 | B 4.3 | B 4.4 | B 4.5 | B 4.6 | B 4.7 | B 4.8 |
|---|---|---|---|---|---|---|---|---|
| Solubilizer A4 | 6% | 10% | 24% | 6% | 4% | 15% | 7% | 8% |
| fatty phase | woody 0.5% | woody 5% | musky 5% | pin ess siberie 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 5.1 | B 5.2 | B 5.3 | B 5.4 | B 5.5 | B 5.6 | B 5.7 | B 5.8 |
|---|---|---|---|---|---|---|---|---|
| Solubilizer A5 | 6% | 10% | 22% | 6% | 3% | 14% | 6% | 7% |
| fatty phase | woody 0.5% | woody 5% | musky 5% | pin ess siberie: 1% | eucalyptus globulus ess chine: 1% | aldehyde cyclamen extra: 1% | lycopene : 0.01% | paprika: 0.05% |
| water | Up to 100% | Up to 100% | Up to 100% | Up to 100% | Up to 100% | Up to 100% | Up to 100% | Up to 100% |

| Compositions | B 6.1 | B 6.2 | B 6.3 | B 6.4 | B 6.5 | B 6.6 | B 6.7 | B 6.8 |
|---|---|---|---|---|---|---|---|---|
| Solubilizer A6 | 2% | 12% | 22% | 4% | 3% | 15% | 5% | 5% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 7.1 | B 7.2 | B 7.3 | B 7.4 | B 7.5 | B 7.6 | B 7.7 | B 7.8 |
|---|---|---|---|---|---|---|---|---|
| Solubilizer A7 | 6% | 10% | 21% | 4% | 3% | 15% | 5% | 5% |
| fatty phase | woody 0.5% | woody 5% | musky 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 8.1 | B 8.2 | B 8.3 | B 8.4 | B 8.5 | B 8.6 | B 8.7 | B 8.8 |
|---|---|---|---|---|---|---|---|---|
| Solubilizer A8 | 2% | 14% | 25% | 6% | 4% | 14% | 12% | 15% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 9.1 | B 9.2 | B 9.3 | B 9.4 | B 9.5 | B 9.6 | B 9.7 | B 9.8 |
|---|---|---|---|---|---|---|---|---|
| Solubilizer A9 | 5% | 19% | 26% | 8% | 4% | 16% | 15% | 17% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 10.1 | B 10.2 | B 10.3 | B 10.4 | B 10.5 | B 10.6 | B 10.7 | B 10.8 |
|---|---|---|---|---|---|---|---|---|
| Solubilizer A10 | 4% | 13% | 20% | 6% | 4% | 14% | 10% | 12% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 11.1 | B 11.2 | B 11.3 | B 11.4 | B 11.5 | B 11.6 | B 11.7 | B 11.8 |
|---|---|---|---|---|---|---|---|---|
| Solubilizer A11 | 2% | 10% | 16% | 4% | 2% | 10% | 5% | 9% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 12.1 | B 12.2 | B 12.3 | B 12.4 | B 12.5 | B 12.6 | B 12.7 | B 12.8 |
|---|---|---|---|---|---|---|---|---|
| Solubilizer A12 | 6% | 13% | 22% | 4% | 3% | 15% | 5% | 5% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 13.1 | B 13.2 | B 13.3 | B 13.4 | B 13.5 | B 13.6 | B 13.7 | B 13.8 |
|---|---|---|---|---|---|---|---|---|
| Solubilizer A13 | 6% | 13% | 22% | 4% | 3% | 15% | 5% | 9% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

| Compositions | B 14.1 | B 14.2 | B 14.3 | B 14.4 | B 14.5 | B 14.6 | B 14.7 | B 14.8 |
|---|---|---|---|---|---|---|---|---|
| Solubilizer A14 | 6% | 13% | 22% | 4% | 3% | 15% | 9% | 10% |
| fatty phase | woody: 0.5% | woody: 5% | musky: 5% | pin ess siberie: 1% | eucalyptus globulus ess chine: 1% | aldehyde cyclamen extra: 1% | lycopene: 0.01% | paprika: 0.05% |
| water | Up to 100% | Up to 100% | Up to 100% | Up to 100% | Up to 100% | Up to 100% | Up to 100% | Up to 100% |

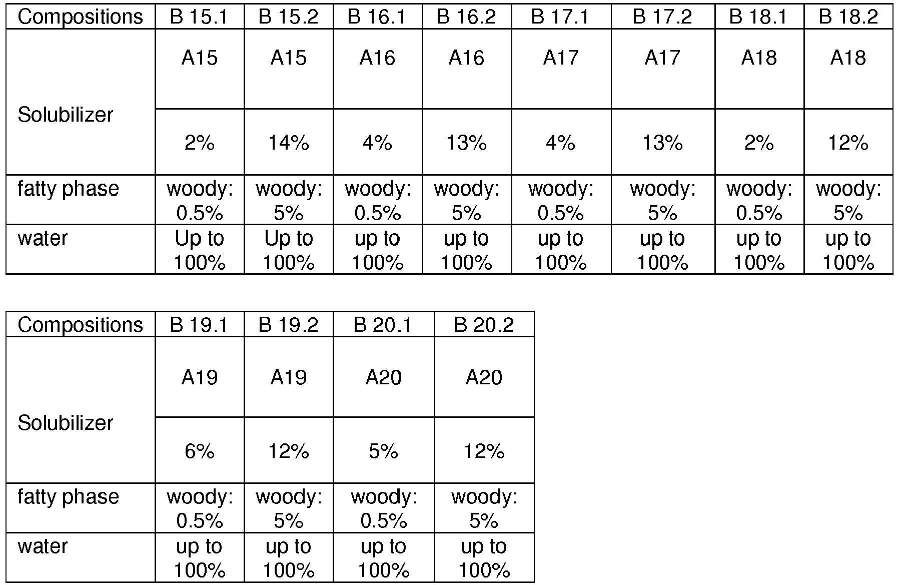

### C. Comparative data

The solubilizers A1 to A14 were compared with four commercialized surfactants for solubilizing a fatty phase in water: Solubilizer 1, Solubilizer 2, Solubilizer 3 and Solubilizer 4.
Solubilizer 1: APG
Solubilizer 2: Polyglyceryl fatty acid esters, lipoaminoacid and APG
Solubilizer 3: Sugar ester
Solubilizer 4 : Cremophor RH40

The following comparative compositions C3 to C26 were prepared according to the protocol disclosed in section B above. The percentages are % by weight relative to the total weight of the composition.

| Compositions | C11 | C12 | C13 | C14 | C15 | C16 | C17 | C18 |
|---|---|---|---|---|---|---|---|---|
| Solubilizer : Solubilizer 1 | 10% | 26% | 26% | 20% | 10% | 20% | 21% | 26% |
| fatty phase | woody : 0.5% | woody: 5% | musky: 5% | pin ess siberie : 1% | eucalyptus globulus ess chine : 1% | ald cyclamen extra : 1% | lycopene : 0.01% | paprika : 0.05% |
| water | Up to 100% | Up to 100% | Up to 100% | Up to 100% | Up to 100% | Up to 100% | Up to 100% | Up to 100% |

| Compositions | C19 | C20 | C21 | C22 | C23 | C24 | C25 | C26 |
|---|---|---|---|---|---|---|---|---|
| Solubilizer : Solubilizer 2 | 13% | 26% | 26% | 21% | 5% | 25% | 26% | 26% |
| fatty phase | woody : 0.5% | woody: 5% | musky: 5% | pin ess siberie : 1% | eucalyptus globulus ess chine : 1% | ald cyclamen extra : 1% | lycopene : 0.01% | paprika : 0.05% |
| water | Up to 100% | Up to 100% | Up to 100% | Up to 100% | Up to 100% | Up to 100% | Up to 100% | Up to 100% |

| Compositions | C3 | C4 | C5 | C6 | C7 | C8 | C9 | C10 |
|---|---|---|---|---|---|---|---|---|
| Solubilizer : Solubilizer 3 | 20% | 26% | 26% | 26% | 25% | 26% | 14% | 17% |
| fatty phase | woody : 0.5% | woody : 5% | musky : 5% | pin ess siberie : 1% | eucalyptus globulus ess chine : 1% | ald cyclamen extra : 1% | lycopene : 0.01% | paprika : 0.05% |
| water | Up to 100% | Up to 100% | Up to 100% | Up to 100% | Up to 100% | Up to 100% | Up to 100% | Up to 100% |

| Compositions | C3 | C4 | C5 | C6 | C7 | C8 | C9 | C10 |
|---|---|---|---|---|---|---|---|---|
| Solubilizer : Solubilizer 4 | 3% | 17% | 20% | 3% | 3% | 3% | 5% | 10% |
| fatty phase | woody : 0.5% | woody : 5% | musky : 5% | pin ess siberie : 1% | eucalyptus globulus ess chine : 1% | ald cyclamen extra : 1% | lycopene : 0.01% | paprika : 0.05% |
| water | Up to 100% | Up to 100% | Up to 100% | Up to 100% | Up to 100% | Up to 100% | Up to 100% | Up to 100% |

These results show that the solubilizers according to the invention are efficient for solubilizing a fatty phase in aqueous compositions, and more particularly are more efficient than solubilizers 1, 2 and 3.

For example, only 2% to 6% by weight, relative to the total weight of the composition, of the solubilizer according to the invention (one of A1 to A14) are necessary for solubilizing 0.5% of argan (see tables in section B, B1.1 to B14.1), whereas 20% by weight of solubilizer 3 (C3), 10% by weight of solubilizer 1 (C11) or 13% by weight of solubilizer 2 (C19) are needed for solubilizing the same amount of fatty phase.

It is advantageous to use a lower amount of solubilizer than conventional solubilizers for solubilizing a fatty phase. Besides, the solubilizers according to the invention advantageously allow solubilizing a higher amount of fatty phase than conventional solubilizers.

Solubilizer 4 is a well-known solubilizer which is efficient but it is an ethoxylated surfactant. Indeed, solubilizer 4 results from petrochemical synthesis and leads to the formation of formaldehyde and 1,4-dioxane (by-products) which are toxic.

The solubilizers according the invention are efficient for solubilizing a fatty phase in aqueous composition, and are advantageously not petrochemical derivatives.

### D. Additional comparative data

### D.1. With compositions comprising only one ingredient

| | | M1 | M2 | M3 | M4 | M5 | M6 | M7 | M8 | M9 | M10 | M11 | M12 | M13 | M14 |
|---|---|---|---|---|---|---|---|---|---|---|---|---|---|---|---|
| Sucrose esters | Sucrose Laurate | 100% | | | | | | | | | | | | | |
| APG | Capryly/Capryl Glucoside | - | 60% | - | - | - | - | - | - | - | - | - | - | - | - |
| | Lauryl glucoside | - | - | 100% | - | - | - | - | - | - | - | - | - | - | - |
| | Hexyl glucoside | - | - | - | 100% | - | - | - | - | - | - | - | - | - | - |
| | Coco glucoside | - | - | - | - | 100% | - | - | - | - | - | - | - | - | - |
| Polyglyceryl esters | Polyglyceryl-6 Laurate | - | - | - | - | - | 100% | - | - | - | - | - | - | - | - |
| | Polyglyceryl-10 Isostearate | - | - | - | - | - | - | 100% | - | - | - | - | - | - | - |
| | Polyglyceryl-4 Caprate | - | - | - | - | - | - | - | 100% | - | - | - | - | - | - |
| Gemini | Sodium dilauramidoglutamide lysine | - | - | - | - | - | - | - | - | 29% | - | - | - | - | - |
| | Sodium bishydroxyethylglycinate lauryl-glucosides crosspolymer | - | - | - | - | - | - | - | - | - | 40% | - | - | - | - |
| | Sodium bishydroxyethylglycinate Coco-glucosides crosspolymer | - | - | - | - | - | - | - | - | - | - | 40% | - | - | - |
| | Sodium didecyl glucosides hydroxy propyl phosphate | - | - | - | - | - | - | - | - | - | - | - | 40% | - | - |
| | Sodium hydroxyplopylphosphate cocoglucoside crosspolymer | - | - | - | - | - | - | - | - | - | - | - | - | 100% | - |
| | poly(sodium dilaurylglucosides Hydroxypropyl phosphate) | - | - | - | - | - | - | - | - | - | - | - | - | - | 40% |
| | water | - | 40% | - | - | - | - | - | - | 71% | 60% | 60% | 60% | - | 60% |
| | Total | 100% | 100% | 100% | 100% | 100% | 100% | 100% | 100% | 100% | 100% | 100% | 100% | 100% | 100% |

The following compositions were prepared by mixing a fatty phase with a composition selected from M1 to M14 at ambient temperature. Then water was added slowly at the beginning, then in larger quantity to obtain the following compositions N.

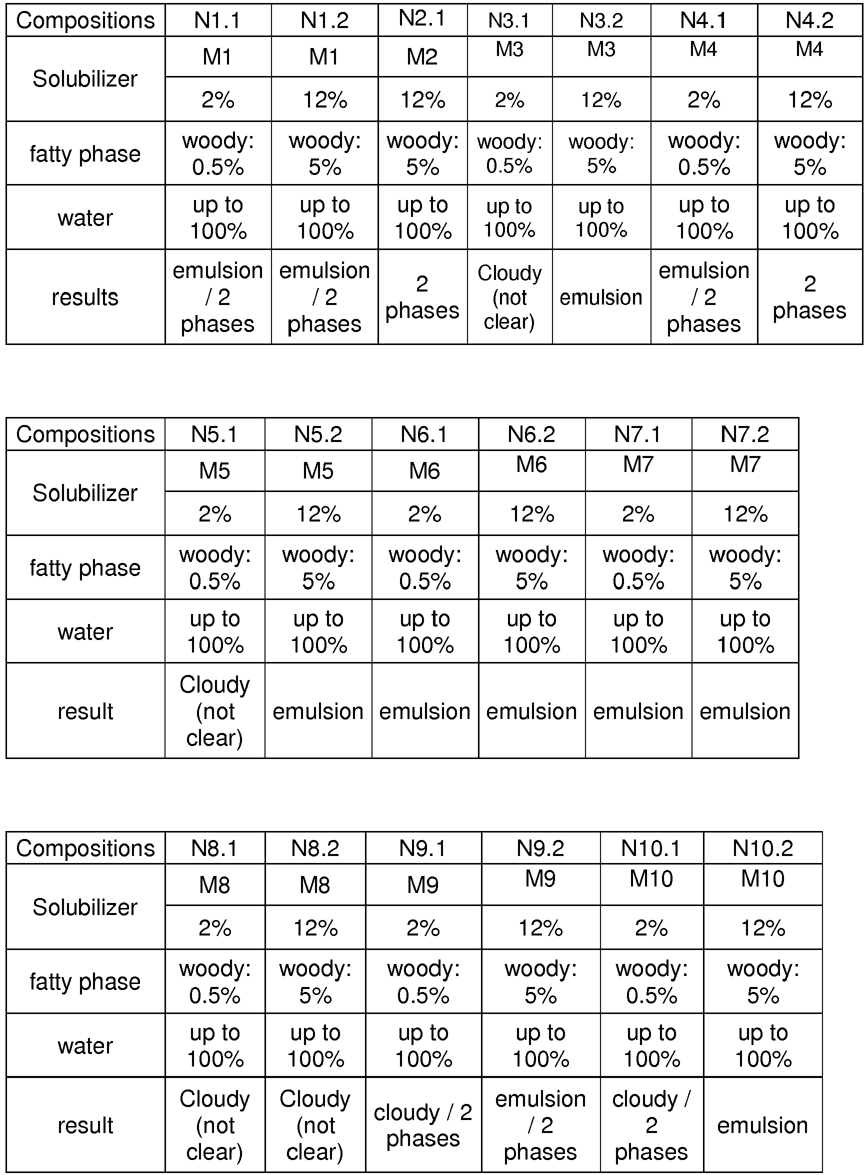

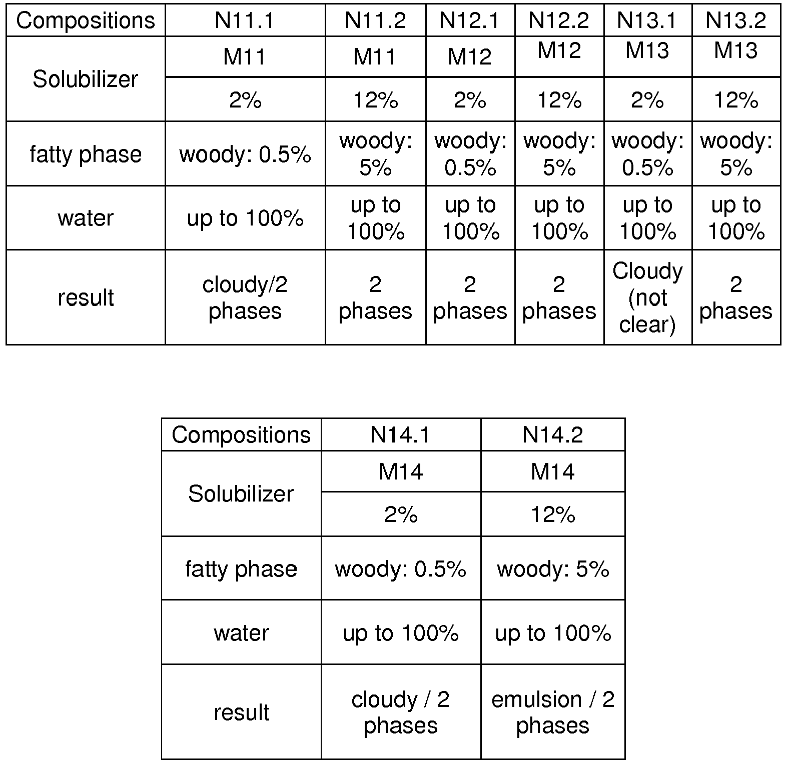

Compositions M2 to M5 correspond to compositions only comprising APG, while compositions M6 to M8 correspond to compositions only comprising polyglycerol fatty acid(s) ester. At last, compositions M9 to M14 correspond to compositions only comprising a gemini surfactant.

These results show that compositions M1 to M14 are not efficient for solubilizing a fatty phase. Indeed, only emulsions (2 phases) or cloudy (not clear) compositions are obtained.

### D.2. With compositions comprising two ingredients

The following aqueous compositions P1 to P21 were prepared as follows:
- Adding the gemini surfactant in a beaker of 250 mL at ambient temperature; then
- Adding the APG surfactant and mixing; then
- Adding the polyglycerol fatty acid(s) ester(s) into the mixture; then
- Mixing at ambient temperature until a transparent composition is obtained.

**Table a: compositions without APG**

| | | P1 | P2 | P3 | P4 | P5 | P6 | P7 | P8 | P9 | P10 | P11 | P12 | P13 | P14 | P15 | P16 |
|---|---|---|---|---|---|---|---|---|---|---|---|---|---|---|---|---|---|
| Polyglycerol fatty acid ester | Polyglyceryl-4 Caprate | 70% | 50% | 30% | 40% | 70% | 50% | 30% | 40% | 70% | 50% | 30% | 40% | 60% | 60% | 80% | 20% |
| | Polyglyceryl-6 Laurate | 10% | 30% | 50% | 20% | 10% | 30% | 50% | 20% | 10% | 30% | 50% | 20% | - | - | - | - |
| Gemini | Sodium dilauramidoglutamide lysine (29% by weight in water) | 20% | 20% | 20% | 40% | - | - | - | - | - | - | - | - | - | - | - | - |
| | Sodium bishydroxyethylglycinate Coco-glucosides crosspolymer (40% by weight in water) | - | - | - | - | 20% | 20% | 20% | 40% | - | - | - | - | - | 40% | - | 80% |
| | Sodium bishydroxyethylglycinate lauryl-glucosides crosspolymer (40% by weight in water) | - | - | - | - | - | - | - | - | 20% | 20% | 20% | 40% | 40% | - | 20% | - |
| | Total | 100% | 100% | 100% | 100% | 100% | 100% | 100% | 100% | 100% | 100% | 100% | 100% | 100% | 100% | 100% | 100% |

**Table b: Compositions without polyglycerol fatty acid ester**

| | | P17 | P18 |
|---|---|---|---|
| APG | Capryly/Capryl Glucoside (60% by weight in water) | 60% | 60% |
| | Hexyl glucoside | - | - |
| | Coco glucoside | - | - |
| Polyglycerol fatty acid ester | Polyglyceryl-4 Caprate | - | - |
| | Polyglyceryl-6 Laurate | - | - |
| Gemini | Sodium dilauramidoglutamide lysine (29% by weight in water) | | |
| | Sodium bishydroxyethylglycinate Coco-glucoside crosspolymer (40% by weight in water) | - | 40% |
| | Sodium bishydroxyethylglycinate lauryl-glucoside crosspolymer (40% by weight in water) | 40% | - |
| | total | 100% | 100% |

**Table c: compositions without gemini**

| | | P19 | P20 | P21 |
|---|---|---|---|---|
| APG | Caprylyl/Capryl Glucoside (60% by weight in water) | 60% | 60% | 60% |
| Polyglycerol fatty acid ester | Polyglyceryl-4 Caprate | 40% | - | 20% |
| | Polyglyceryl-6 Laurate | - | 40% | 20% |
| | total | 100% | 100% | 100% |

The following compositions were prepared by mixing a fatty phase with a composition selected from P1 to P21 at ambient temperature. Then water was added slowly at the beginning, then in larger quantity to obtain the following compositions Q.

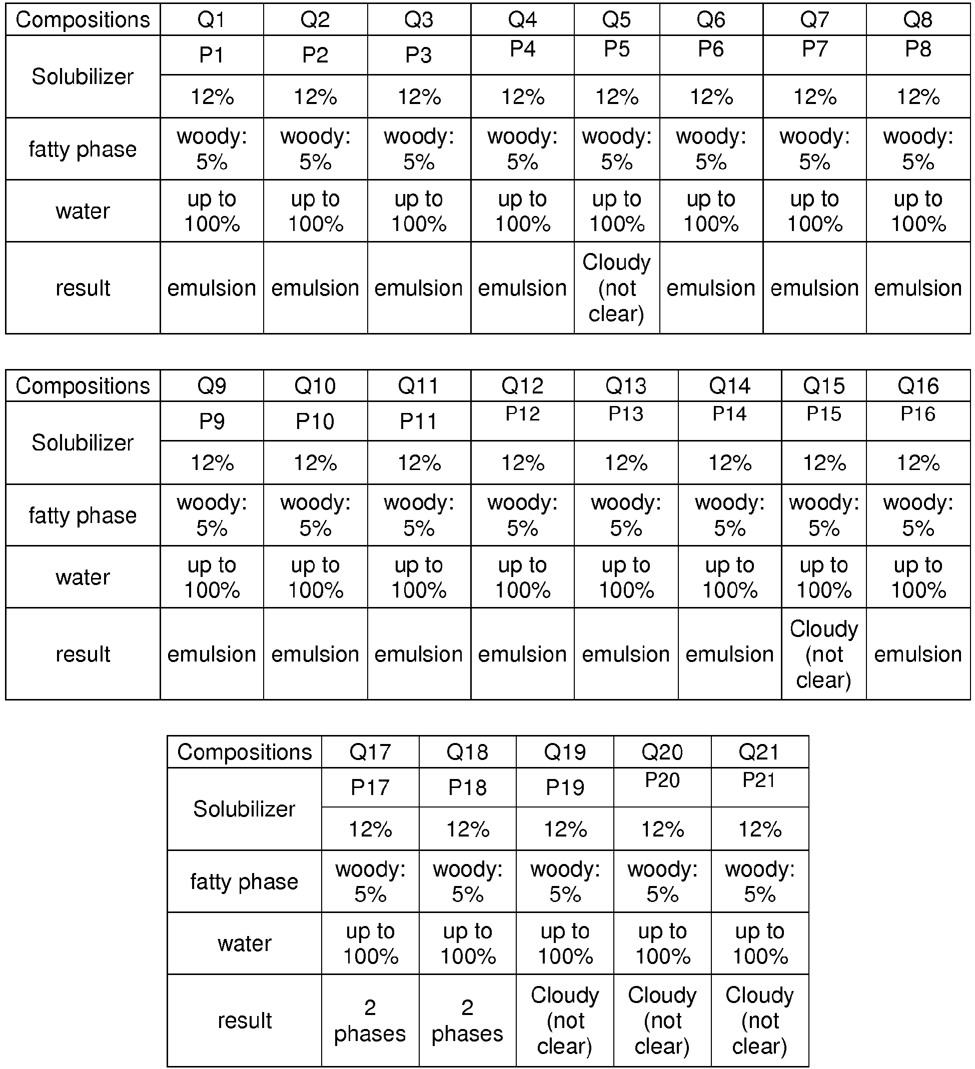

Compositions P1 to P16 correspond to compositions comprising a gemini and polyglycerol fatty acid ester, while compositions P17 to P18 correspond to compositions comprising a gemini surfactant and an APG. At last, compositions P19 to P21 correspond to compositions comprising an APG and polyglycerol fatty acid ester.

These results show that compositions P1 to P21 are not efficient for solubilizing a fatty phase. Indeed, only emulsions (2 phases) or cloudy (not clear) compositions are obtained.

## Claims

1. Composition comprising:
a) at least one gemini surfactant;
b) at least one alkylpolyglucoside; and
c) at least one polyglycerol fatty acid(s) ester, wherein the fatty acid comprises from 4 to 36 carbon atoms,
wherein said composition comprises from 0.1% to 20% by weight of gemini surfactant(s) based on the total weight of the composition, and
wherein said gemini surfactant has the following formula (A): 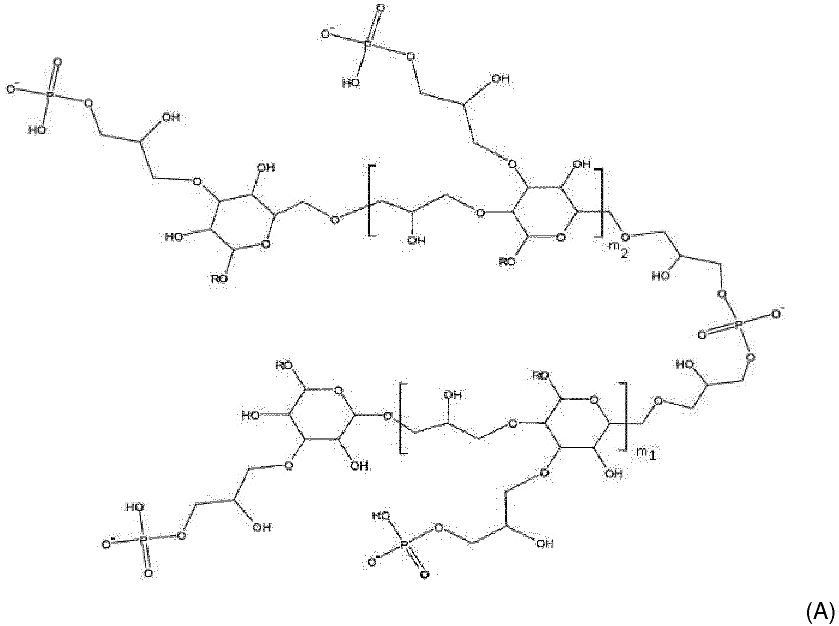 wherein:
- R represents a linear or branched alkyl group comprising from 1 to 26 carbon atoms,
- Z is H⁺ or Na⁺,
- m₁ and m₂ are, independently of each other, integer comprised between 1 and 20, and
- y is an integer wherein y = m₁ + m₂ + 3;
or wherein the gemini surfactant has the following formula (I-1): 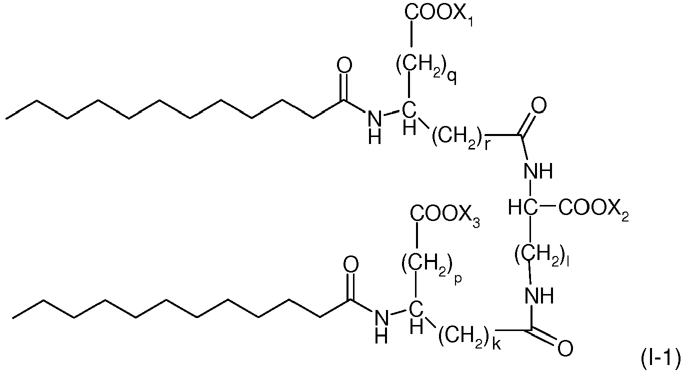 or their diastereoisomers,
- X₁ is H or M, M being an alkali metal or alkaline-earth metal,
- X2 is H or M, M being as defined above,
- X3 is H or M, M being as defined above, and
- r, q, l, p, and k are integers comprised between 0 and 10.

2. Composition according to claim 1, wherein the gemini surfactant has the following formula (I-2): 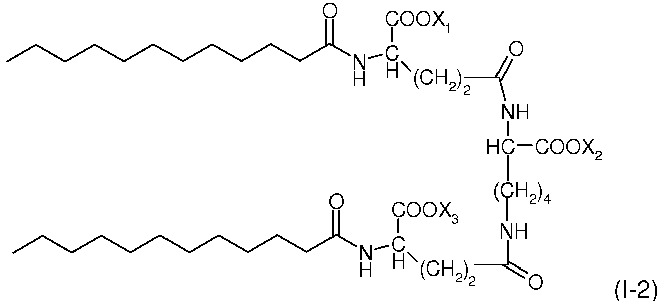 or their diastereoisomers, X₁, X₂ and X₃ being as defined in claim 1.

3. Composition according to claim 2, wherein the gemini surfactant is sodium dilauramidoglutamide lysine.

4. Composition according to claim 1, wherein the gemini surfactant is selected from the group consisting of: sodium hydroxypropylphosphate decylglucoside crosspolymer, sodium hydroxypropylphosphate laurylglucoside crosspolymer, sodium hydroxypropylphosphate cocoglucoside crosspolymer, sodium didecylglucoside hydroxypropylphosphate, poly(sodium dilaurylglucoside hydroxypropylphosphate), poly(sodium decylglucoside hydroxypropylphosphate), poly(sodium laurylglucoside hydroxypropylphosphate), poly(sodium cocoylglucoside hydroxypropylphosphate), and mixtures thereof.

5. Composition according to any one of claims 1 to 4, wherein the alkylpolyglucoside has the following formula (IV):
Rₐ(O)(G)ₓ (IV)
wherein:
- Rₐ represents a linear or branched alkyl group comprising from 2 to 22 carbon atoms;
- G represents a sugar moiety comprising 5 or 6 carbon atoms; and
- X is an integer comprised between 1 and 15.

6. Composition according to claim 5, wherein the alkylpolyglucoside is selected from the group consisting of: cocoglucoside, decyl glucoside, lauryl glucoside, caprylyl/capryl glucoside, arachidyl glucoside, butyl glucoside, caprylyl glucoside, cetearyl glucoside, coco glucoside, ethyl glucoside, hexadecyl D-glucoside, isostearyl glucoside, lauryl glucoside, myristyl glucoside, octadecyl D-glucoside, octyldodecyl glucoside, undecyl glucoside, and their mixtures.

7. Composition according to any one of claims 1 to 6, wherein the polyglycerol fatty acid(s) ester is selected from the group consisting of: polyglyceryl-4 caprylate/caprate, polyglyceryl-5 caprylate/caprate, polyglyceryl-6 caprylate/caprate, polyglyceryl-7 caprylate/caprate, polyglyceryl-8 caprylate/caprate, polyglyceryl-9 caprylate/caprate, polyglyceryl-10 caprylate/caprate, polyglyceryl-3 caprate, polyglyceryl-4 caprate, polyglyceryl-5 caprate, polyglyceryl-6 caprate, polyglyceryl-7 caprate, polyglyceryl-8 caprate, polyglyceryl-9 caprate, polyglyceryl-10 caprate, polyglyceryl-10 decastearate, polyglyceryl-3 diisostearate, polyglyceryl-10 diisostearate, polyglyceryl-4 laurate, polyglyceryl-5 laurate, polyglyceryl-6 laurate, polyglyceryl-7 laurate, polyglyceryl-8 laurate, polyglyceryl-9 laurate, polyglyceryl-10 laurate, polyglyceryl-3 cocoate, polyglyceryl-4 cocoate, polyglyceryl-6 cocoate, polyglyceryl-7 cocoate, polyglyceryl-8 cocoate, polyglyceryl-9 cocoate, polyglyceryl-10 cocoate, polyglyceryl-11 cocoate, polyglyceryl-12 cocoate, polyglyceryl-6 myristate, polyglyceryl-7 myristate, polyglyceryl-8 myristate, polyglyceryl-9 myristate, polyglyceryl-10 myristate, polyglyceryl-11 myristate, polyglyceryl-12 myristate, polyglyceryl-10 oleate, polyglyceryl-11 oleate, polyglyceryl-12 oleate, polyglyceryl-3 palmitate, polyglyceryl-10 palmitate, polyglyceryl-3 polyricinoleate, polyglyceryl-6 polyricinoleate, polyglyceryl-10 polyricinoleate polyglyceryl-3 stearate, polyglyceryl-10 stearate, polyglyceryl-11 stearate, polyglyceryl-12 stearate, polyglyceryl 10-isostearate, and mixtures thereof.

8. Composition according to any one of claims 1 to 7, further comprising at least one sugar fatty acid(s) ester.

9. Composition according to any one of claims 1 to 8, comprising:
a) from 0.5% to 10% by weight of gemini surfactant(s);
b) from 25% to 50% by weight of alkylpolyglucoside(s);
c) from 2% to 50% by weight of polyglycerol fatty acid(s) ester(s);
d) from 0% to 8% by weight of sugar fatty acid(s) ester(s); and
e) from 0% to 10% by weight of pentylene glycol;
based on the total weight of the composition.

10. An aqueous composition comprising:
- an aqueous phase;
- the composition according to any one of claims 1 to 9; and
- a fatty phase.

11. The aqueous composition according to claim 10, wherein the fatty phase comprises at least one compound selected from the group consisting of: coloring agents, odorous substances of synthetic origin, fragrance aromatic compounds, fatty acids, fatty acid esters, fatty alcohols, vitamins, natural extracts, oils, perfumes, UV filters and their mixtures.

12. The aqueous composition according to any one of claims 10 or 11, being a perfume, eau de toilette, a clear deodorant, a moisturizing lotion, a moisturizing spray, a micellar water, a cleansing lotion, a cleansing solution gel, a two phase cleansing lotion, a cleansing scrub gel, a remover such as a rinse off remover or leave on remover, an after shave alcohol free, a purifying water with essential oils, a D phase system, a natural sprayable hair conditioner, a wet wipe solution, a natural body splash, a styling gel, an antidandruff lotion preparation, an UV sunscreen, a bath preparation, a hair care preparation, or a make-up preparation skin care product, sun care product, hair care products, make-up product, or skin care product.

## Patentansprüche

1. Zusammensetzung aufweisend:
a) mindestens ein Gemini-Tensid;
b) mindestens ein Alkylpolyglucosid; und
c) mindestens einen Polyglycerinfettsäureester, wobei die Fettsäure 4 bis 36 Kohlenstoffatome aufweist,
wobei die Zusammensetzung von 0,1 Gew.-% bis 20 Gew.-% Gemini-Tensid(e) basierend auf dem Gesamtgewicht der Zusammensetzung aufweist, und
wobei das Gemini-Tensid die folgende Formel (A) aufweist: 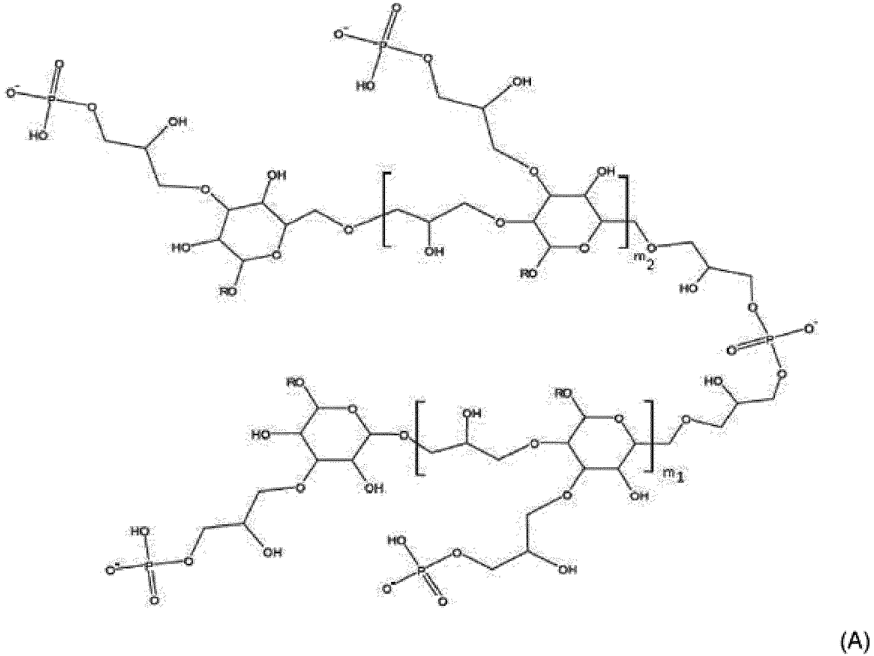 wobei:
- R eine lineare oder verzweigte Alkylgruppe mit 1 bis 26 Kohlenstoffatomen darstellt,
- Z H⁺ oder Na⁺ ist,
- m₁ und m₂ unabhängig voneinander eine ganze Zahl zwischen 1 und 20 sind, und
- y eine ganze Zahl ist, wobei y = m₁ + m₂ + 3;
oder wobei das Gemini-Tensid die folgende Formel (I-1) aufweist: 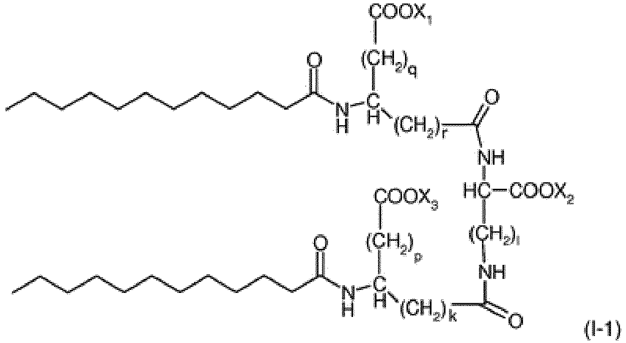 oder dessen Diastereoisomere,
wobei
- X₁ H oder M ist, wobei M ein Alkalimetall oder Erdalkalimetall ist
- X₂ H oder M ist, wobei M wie oben definiert ist,
- X₃ H oder M ist, wobei M wie oben definiert ist, und
- r, q, l, p und k ganze Zahlen zwischen 0 und 10 sind.

2. Zusammensetzung nach Anspruch 1, wobei das Gemini-Tensid die folgende Formel (I-2) aufweist: 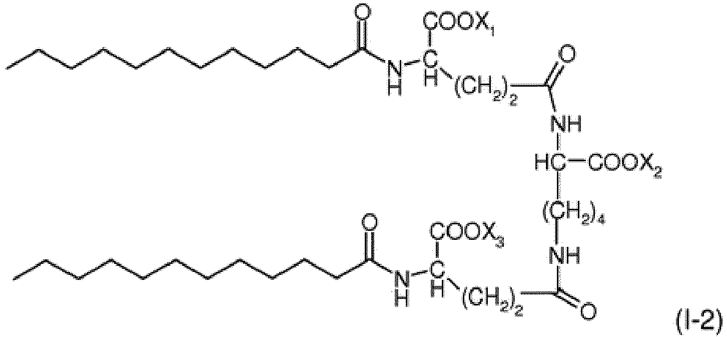 oder dessen Diastereoisomere,
wobei X₁, X₂ und X₃ wie in Anspruch 1 definiert sind.

3. Zusammensetzung nach Anspruch 2, wobei das Gemini-Tensid Natriumdilauramidoglutamidlysin ist.

4. Zusammensetzung nach Anspruch 1, wobei das Gemini-Tensid ausgewählt ist aus der Gruppe bestehend aus: Natriumhydroxypropylphosphat-Decylglucosid-Crosspolymer, Natriumhydroxypropylphosphat-Laurylglucosid-Crosspolymer, Natriumhydroxypropylphosphat-Cocoglucosid-Crosspolymer, Natriumdodecylglucosidhydroxypropylphosphat, Poly (natriumdilaurylglucosidhydroxypropylphosphat), Poly (natriumdecylglucosidhydroxypropylphosphat), Poly (natriumlaurylglucosidhydroxypropylphosphat), Poly (natriumcocoylglucosidhydroxypropylphosphat) und Mischungen davon.

5. Zusammensetzung nach irgendeinem der Ansprüche 1 bis 4, wobei das Alkylpolyglucosid die folgende Formel (IV) aufweist:
Rₐ(O)(G)ₓ (IV)
wobei:
- Rₐ eine lineare oder verzweigte Alkylgruppe mit 2 bis 22 Kohlenstoffatomen darstellt,
- G eine Zuckereinheit mit 5 oder 6 Kohlenstoffatomen darstellt; und
- X eine ganze Zahl zwischen 1 und 15 ist.

6. Zusammensetzung nach Anspruch 5, wobei das Alkylpolyglucosid ausgewählt ist aus der Gruppe bestehend aus: Cocoglucosid, Decylglucosid, Laurylglucosid, Caprylyl / Caprylglucosid, Arachidylglucosid, Butylglucosid, Caprylylglucosid, Cetearylglucosid, Cocoglucosid, Ethylglucosid, Hexadecyl-D-Glucosid, Isostearylglucosid, Laurylglucosid, Myristylglucosid, Octadecyl-D-glucosid, Octyldodecylglucosid, Undecylglucosid und deren Mischungen.

7. Zusammensetzung nach irgendeinem der Ansprüche 1 bis 6, wobei der Polyglycerinfettsäureester ausgewählt ist aus der Gruppe bestehend aus: Polyglyceryl-4-Caprylat / Caprat, Polyglyceryl-5-Caprylat / Caprat, Polyglyceryl-6-Caprylat / Caprat, Polyglyceryl-7-Caprylat / Caprat, Polyglyceryl-8-Caprylat / Caprat, Polyglyceryl-9-Caprylat / Caprat, Polyglyceryl-10-Caprylat / Caprat, Polyglyceryl-3 Caprat, Polyglyceryl-4 Caprat, Polyglyceryl-5-Caprat, Polyglyceryl-6 Caprat, Polyglyceryl-7-Caprat, Polyglyceryl-8-Caprat, Polyglyceryl-9-Caprat, Polyglyceryl-10-Caprat, Polyglyceryl-10-Decastearat, Polyglyceryl-3-Diisostearat, Polyglyceryl-10-Diisostearat, Polyglyceryl-4-Laurat, Polyglyceryl-5-Laurat, Polyglyceryl-6-Laurat, Polyglyceryl-7-Laurat, Polyglyceryl-8-Laurat, Polyglyceryl-9-Laurat, Polyglyceryl-10-Laurat, Polyglyceryl-3-Cocoat, Polyglyceryl-4-Cocoat, Polyglyceryl-6-Cocoat, Polyglyceryl-7-Cocoat, Polyglyceryl-8-Cocoat, Polyglyceryl-9-Cocoat, Polyglyceryl-10-Cocoate, Polyglyceryl-11-Cocoat, Polyglyceryl-12-Cocoat, Polyglyceryl-6-Myristat, Polyglyceryl-7-Myristat, Polyglyceryl-8-Myristat, Polyglyceryl-9-Myristat, Polyglyceryl-10-Myristat, Polyglyceryl-11-Myristat, Polyglyceryl-12-Myristat; Polyglyceryl-10-Oleat, Polyglyceryl-11-Oleat, Polyglyceryl-12-Oleat, Polyglyceryl-3-Palmitat, Polyglyceryl-10-Palmitat, Polyglyceryl-3-Polyricinoleat, Polyglyceryl-6-Polyricinoleat, Polyglyceryl-10-Polyricinoleat, Polyglyceryl-3-Stearat, Polyglyceryl-10-Stearat, Polyglyceryl-11-Stearat, Polyglyceryl-12-Stearat, Polyglyceryl-10-Isostearat und Mischungen davon.

8. Zusammensetzung nach irgendeinem der Ansprüche 1 bis 7 ferner aufweisend mindestens einen Zuckerfettsäureester.

9. Zusammensetzung nach irgendeinem der Ansprüche 1 bis 8 aufweisend:
a) 0,5 bis 10 Gew .-% Gemini-Tensid (e);
b) 25 bis 50 Gew .-% Alkylpolyglucosid (e);
c) 2 bis 50 Gew .-% Polyglycerinfettsäureester;
d) 0 bis 8 Gew .-% Zuckerfettsäureester; und
e) 0 bis 10 Gew .-% Pentylenglykol;
basierend auf dem Gesamtgewicht der Zusammensetzung.

10. Wässrige Zusammensetzung aufweisend:
- eine wässrige Phase;
- die Zusammensetzung nach einem der Ansprüche 1 bis 9; und
- eine Fettphase.

11. Wässrige Zusammensetzung nach Anspruch 10, wobei die Fettphase mindestens eine Verbindung aufweist ausgewählt aus der Gruppe bestehend aus: Farbmitteln, Riechstoffen von synthetischem Ursprung, Duftaromaten, Fettsäuren, Fettsäureestern, Fettalkoholen, Vitaminen, natürlichen Extrakten, Ölen, Düften, UV-Filtern und deren Mischungen.

12. Wässrige Zusammensetzung nach einem der Ansprüche 10 oder 11, die ein Parfüm, ein Eau de Toilette, ein klares Deodorant, eine Feuchtigkeitslotion, ein Feuchtigkeitsspray, ein mizellares Wasser, eine Reinigungslotion, ein Reinigungslösungsgel, eine Zweiphasenreinigungslotion, ein Reinigungs-Peeling-Gel, ein Entferner wie ein Abspül- oder Einwirk-Entferner, ein alkoholfreies After Shave, ein Reinigungswasser mit ätherischen Ölen, ein D-Phasensystem, ein natürliches sprühbares Haarpflegemittel, eine Feuchttuchlösung, ein natürliches Spritzwasser, ein Styling-Gel, ein Antischuppenlotion-Präparat, ein UV-Sonnenschutzmittel, ein Badezusatz, ein Haarpflege-Präparat oder ein Make-up-Präparat, ein Hautpflegprodukt, ein Sonnenschutzprodukt, Haarpflegeprodukte, ein Make-up-Produkt oder Hautpflegeprodukt ist.

## Revendications

1. Composition comprenant :
a) au moins un tensioactif jumelé ;
b) au moins un alkylpolyglucoside ; et
c) au moins un ester d'acide gras et de polyglycérol, dans lequel l'acide gras comprend de 4 à 36 atomes de carbone,
laquelle composition comprend de 0,1 % à 20 % en poids d'un ou plusieurs tensioactifs jumelés par rapport au poids total de la composition, et
dans laquelle ledit tensioactif jumelé répond à la formule (A) suivante : 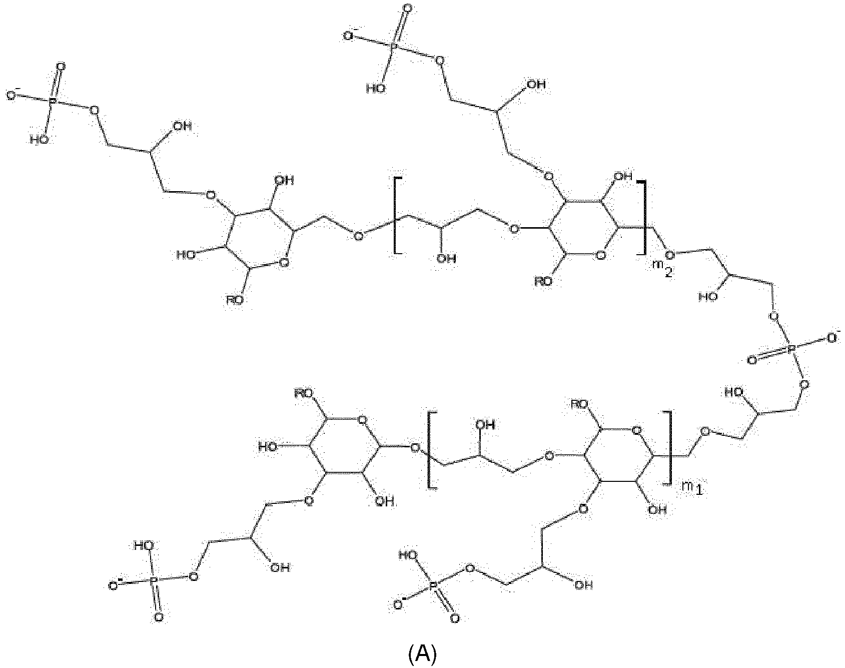 dans laquelle :
- R représente un groupe alkyle linéaire ou ramifié comprenant de 1 à 26 atomes de carbone,
- Z est H⁺ ou Na⁺,
- m₁ et m₂, indépendamment l'un de l'autre, sont des entiers compris entre 1 et 20, et
- y est un entier, avec y = m₁ + m₂ + 3 ;
ou dans laquelle ledit tensioactif jumelé répond à la formule (I-1) suivante : 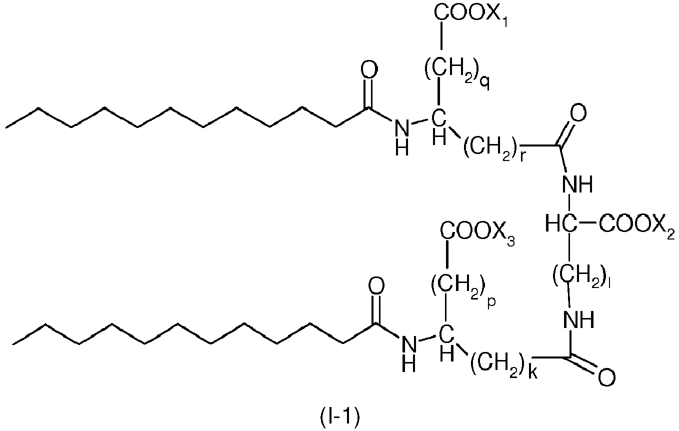 ou ses diastéréoisomères,
- X₁ est H ou M, M étant un métal alcalin ou un métal alcalino-terreux,
- X₂ est H ou M, M étant tel que défini ci-dessus,
- X₃ est H ou M, M étant tel que défini ci-dessus, et
- r, q, l, p et k sont des entiers compris entre 0 et 10.

2. Composition selon la revendication 1, dans laquelle le tensioactif jumelé répond à la formule (I-2) suivante : 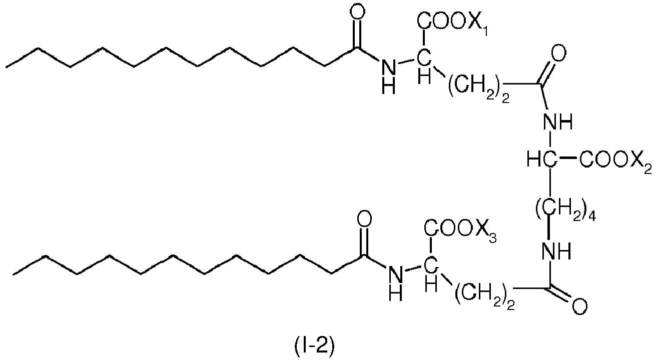 ou ses diastéréoisomères, X₁, X₂ et X₃ étant tels que définis dans la revendication 1.

3. Composition selon la revendication 2, dans laquelle le tensioactif jumelé est la dilauramidoglutamide-lysine sodique.

4. Composition selon la revendication 1, dans laquelle le tensioactif jumelé est choisi dans l'ensemble constitué par : un polymère réticulé d'hydroxypropylphosphate de sodium et de décylglucoside, un polymère réticulé d'hydroxypropylphosphate de sodium et de laurylglucoside, un copolymère réticulé d'hydroxypropylphosphate de sodium et de (coprah-yl)glucoside, l'hydroxypropylphosphate de didécylglucoside sodique, le poly(hydroxypropylphosphate de dilaurylglucoside sodique), le poly(hydroxypropylphosphate de décylglucoside sodique), le poly(hydroxypropylphosphate de laurylglucoside sodique), le poly(hydroxypropylphosphate de (coprah-yl)glucoside sodique), et leurs mélanges.

5. Composition selon l'une quelconque des revendications 1 à 4, dans laquelle l'alkylpolyglucoside répond à la formule (IV) suivante :
Rₐ(O)(G)ₓ (IV)
dans laquelle :
- Rₐ représente un groupe alkyle linéaire ou ramifié comprenant de 2 à 22 atomes de carbone ;
- G représente un fragment sucre comprenant 5 ou 6 atomes de carbone ; et
- X est un entier compris entre 1 et 15.

6. Composition selon la revendication 5, dans laquelle l'alkylpolyglucoside est choisi dans l'ensemble constitué par : le (coprah-yl)glucoside, le décylglucoside, le laurylglucoside, le caprylyl/caprylglucoside, l'arachidylglucoside, le butylglucoside, le caprylylglucoside, le cétéarylglucoside, le (coprah-yl)glucoside, l'éthylglucoside, l'hexadécyl-D-glucoside, l'isostéarylglucoside, le laurylglucoside, le myristylglucoside, l'octadécyl-D-glucoside, l'octyldodécylglucoside, l'undécylglucoside, et leurs mélanges.

7. Composition selon l'une quelconque des revendications 1 à 6, dans laquelle l'ester d'acide gras et de polyglycérol est choisi dans l'ensemble constitué par : le caprylate/caprate de polyglycéryle-4, le caprylate/caprate de polyglycéryle-5, le caprylate/caprate de polyglycéryle-6, le caprylate/caprate de polyglycéryle-7, le caprylate/caprate de polyglycéryle-8, le caprylate/caprate de polyglycéryle-9, le caprylate/caprate de polyglycéryle-10, le caprate de polyglycéryle-3, le caprate de polyglycéryle-4, le caprate de polyglycéryle-5, le caprate de polyglycéryle-6, le caprate de polyglycéryle-7, le caprate de polyglycéryle-8, le caprate de polyglycéryle-9, le caprate de polyglycéryle-10, le décastéarate de polyglycéryle-10, le diisostéarate de polyglycéryle-3, le diisostéarate de polyglycéryle-10, le laurate de polyglycéryle-4, le laurate de polyglycéryle-5, le laurate de polyglycéryle-6, le laurate de polyglycéryle-7, le laurate de polyglycéryle-8, le laurate de polyglycéryle-9, le laurate de polyglycéryle-10, le (coprahyl)ate de polyglycéryle-3, le (coprah-yl)ate de polyglycéryle-4, le (coprah-yl)ate de polyglycéryle-6, le (coprah-yl)ate de polyglycéryle-7, le (coprah-yl)ate de polyglycéryle-8, le (coprah-yl)ate de polyglycéryle-9, le (coprah-yl)ate de polyglycéryle-10, le (coprah-yl)ate de polyglycéryle-11, le (coprah-yl)ate de polyglycéryle-12, le myristate de polyglycéryle-6, le myristate de polyglycéryle-7, le myristate de polyglycéryle-8, le myristate de polyglycéryle-9, le myristate de polyglycéryle-10, le myristate de polyglycéryle-11, le myristate de polyglycéryle-12, l'oléate de polyglycéryle-10, l'oléate de polyglycéryle-11, l'oléate de polyglycéryle-12, le palmitate de polyglycéryle-3, le palmitate de polyglycéryle-10, le polyricinoléate de polyglycéryle-3, le polyricinoléate de polyglycéryle-6, le polyricinoléate de polyglycéryle-10, le stéarate de polyglycéryle-3, le stéarate de polyglycéryle-10, le stéarate de polyglycéryle-11, le stéarate de polyglycéryle-12, l'isostéarate de polyglycéryle-10, et leurs mélanges.

8. Composition selon l'une quelconque des revendications 1 à 7, comprenant en outre au moins un ester d'acide gras et de sucre.

9. Composition selon l'une quelconque des revendications 1 à 8, comprenant :
a) de 0,5 % à 10 % en poids d'un ou plusieurs tensioactifs jumelés ;
b) de 25 % à 50 % en poids d'un ou plusieurs alkylpolyglucosides ;
c) de 2 % à 50 % en poids d'un ou plusieurs esters d'acide gras et de polyglycérol ;
d) de 0 % à 8 % en poids d'un ou plusieurs esters d'acide gras et de sucre ; et
e) de 0 % à 10 % en poids de pentylèneglycol ;
par rapport au poids total de la composition.

10. Composition aqueuse comprenant :
- une phase aqueuse ;
- la composition de l'une quelconque des revendications 1 à 9 ; et
- une phase grasse.

11. Composition aqueuse selon la revendication 10, dans laquelle la phase grasse comprend au moins un composé choisi dans l'ensemble constitué par : les agents colorants, les substances odorantes d'origine synthétique, les composés aromatiques de parfum, les acides gras, les esters d'acide gras, les alcools gras, les vitamines, les extraits naturels, les huiles, les parfums, les filtres UV et leurs mélanges.

12. Composition aqueuse selon l'une quelconque des revendications 10 et 11, qui est un parfum, une eau de toilette, un déodorant transparent, une lotion hydratante, un spray hydratant, une eau micellaire, une lotion nettoyante, un gel de solution nettoyante, une lotion nettoyante biphasique, un gel exfoliant nettoyant, un démaquillant tel qu'un démaquillant à rincer ou un démaquillant sans rinçage, un après-rasage sans alcool, une eau purifiante avec des huiles essentielles, un système en phase D, un après-shampooing à pulvériser naturel, une solution pour lingettes humides, un tonique naturel pour le corps, un gel coiffant, une préparation de lotion antipelliculaire, un écran solaire anti-UV, une préparation pour le bain, une préparation pour le soin des cheveux, ou un produit de soin de la peau pour préparation au maquillage, un produit de soin solaire, un produit de soin des cheveux, un produit de maquillage, ou un produit de soin de la peau.
